# EUROPEAN PATENT APPLICATION

(11) **EP 1 393 747 A1**
(43) Date of publication of application: **03.03.2004**
(21) Application number: 01921830.4
(22) Date of filing: 13.04.2001
(51) Int. Cl.: A61K 45/00, C07D 495/04, A61K 31/519, A61K 31/4365, A61P 25/28

(54) **PREVENTIVES/REMEDIES FOR ALZHEIMER'S DISEASE**

(30) Priority: 13.04.2000 JP 2000112046
(71) Applicant: Takeda Chemical Industries, Ltd., Osaka-shi, Osaka 541-0045 (JP)
(72) Inventor: FURUYA, Shuichi, Tsukuba-shi, Ibaraki 305-0821 (JP); SUZUKI, Nobuhiro, Osaka, 562-0001 (JP)
(74) Representative: von Kreisler, Alek, Dipl.-Chem.
(86) International application number: PCT/JP2001/003189
(87) International publication number: WO 2001/078780

(57) **Abstract**

The present invention provides an agent for the prophylaxis or treatment of Alzheimer's disease. The agent for the prophylaxis or treatment of Alzheimer's disease of the present invention containing a compound having a GnRH antagonistic action shows low toxicity and has a superior preventive and therapeutic effect on Alzheimer's disease.

## Description

### Technical Field

The present invention relates to an agent for the prophylaxis or treatment of Alzheimer's disease.

### Background Art

As the aging of the society proceeds and dementia is increasingly observed, Alzheimer's disease, among others, is becoming a problem. An investigation into the cause and treatment methods thereof are therefore desired.

In a report on a clinical test suggesting the correlation between intracerebral LH, FSH concentrations and Alzheimer's disease (The Journal of Neuroendocrinology, April, 2000, 12(4), 351-4), the findings are shown that the LH, FSH concentrations in the patients of Alzheimer's disease are twice higher than those of non-patients, and that Leuplin lowers the LH, FSH concentrations and inhibits the progress of Alzheimer's disease.

There has not been a report on a pharmaceutical product clinically fully satisfactory for use in a method for the prophylaxis or treatment bf Alzheimer's disease.

### Disclosure of the Invention

The present inventors have found that compounds having various gonadotropin releasing hormone (GnRH)-antagonistic actions lower the concentration(s) of LH and/or FSH, and therefore, they-can be effectively used for the prophylaxis or treatment of Alzheimer's disease. Further studies based on the finding have resulted in the completion of the present invention. Accordingly, the present invention relates to
(1) an agent for the prophylaxis or treatment of Alzheimer's disease, which comprises a compound having a GnRH antagonistic action;
(2) the agent described in the aforementioned (1), wherein the compound is a non-peptide compound;
(3) the agent described in the aforementioned (1), wherein the compound is a fused heterocyclic compound:
(4) the agent described in the aforementioned (1), wherein the compound is represented by the formula: 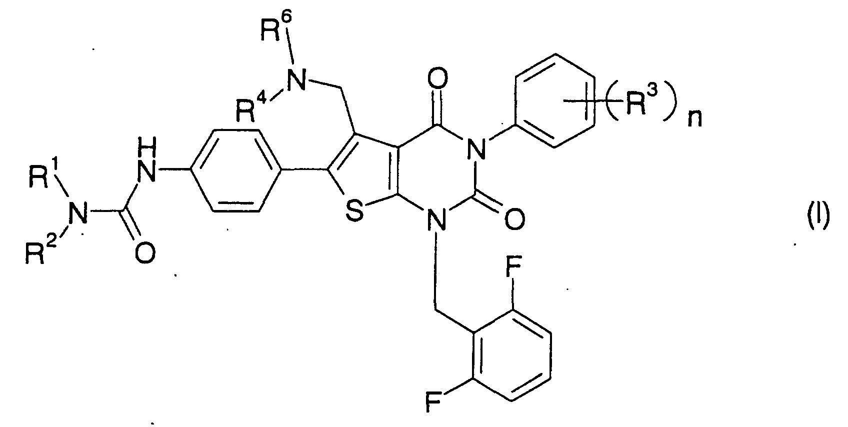 wherein R¹ and R² each represents a hydrogen atom, a hydroxy group, a C₁₋₄ alkoxy group, a C₁₋₄ alkoxy-carbonyl group or a C₁₋₄ alkyl group which may be substituted;
   R³ represents a hydrogen atom, a halogen atom, a hydroxy group or a C₁₋₄ alkoxy group which may be substituted; or adjacent two R³ may form, taken together, a C₁₋₄ alkylenedioxy group; .
   R⁴ represents a hydrogen atom or a C₁₋₄ alkyl group;
   R⁶ represents a C₁₋₄ alkyl group which may be substituted or a group of the formula: 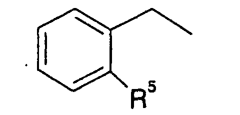 wherein R⁵ represents a hydrogen atom or R⁴ and R⁵ may form, taken together, a heterocycle; and
   n represents an integer of 0 to 5; or a salt thereof [hereinafter sometimes referred to briefly as compound (I)];
(5) the agent described in the aforementioned (1), wherein the compound is 5-(N-benzyl-N-methylaminomethyl)-1-(2,6-difluorobenzyl)-6-[4-(3-methoxyureido)phenyl]-3-phenylthieno-[2,3-d]pyrimidine-2,4(1H,3H)-dione or a salt thereof;
(6) the agent described in the aforementioned (1), wherein the compound is represented by the formula: 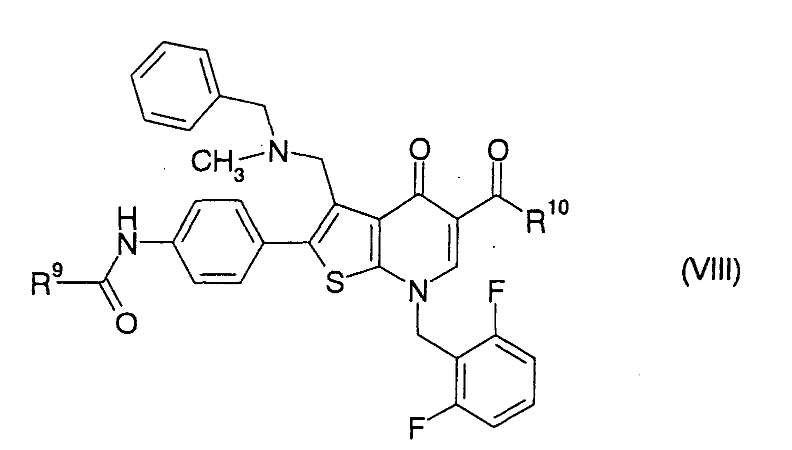 wherein R⁹ represents a C₁₋₇ alkyl group which may be substituted, a C₃₋₇ cycloalkyl group which may be substituted,
   a C₁₋₆ alkoxyamino group which may be substituted or a hydroxyamino group which may be substituted; and
   R¹⁰ represents a C₁₋₇ alkyl group which may be substituted or a phenyl group which may be substituted;
   when R⁹ is an unsubstituted C₁₋₇ alkyl group, then R¹⁰ is a substituted C₁₋₇ alkyl group or substituted phenyl,
   or a salt thereof [hereinafter sometimes referred to briefly as compound (VIII)];
(7) the agent described in the aforementioned (1), wherein the compound is 3-(N-benzyl-N-methylaminomethyl)-4,7-dihydro-5-isobutyryl-7-(2,6-difluorobenzyl)-2-[4-[(1-hydroxycyclopropyl)-carbonylamino]phenyl]-4-oxothieno[2,3-b]pyridine or a salt thereof;
(8) an agent for the prophylaxis or treatment of Alzheimer's disease, which comprises a non-peptide compound that decreases LH and/or RH;
(9) an agent for the prophylaxis or treatment of Alzheimer's disease, which comprises a non-peptide compound that decreases LH and RH; and the like.

### Brief Description of the Drawings

Fig. 1 shows a % LH concentration in the plasma of test monkey, wherein, in the Figure, ■ shows a control (1), ◆ shows control (2), □ shows control (3), Δ shows compound (1) and ▲ shows compound (2) respectively.
Fig. 2 shows a % LH concentration in the plasma of test monkey, wherein, in the Figure, -▲- shows a control group-1, - ◆- shows a control group-2, -Δ- shows a compound administration group-1, -□- shows a compound administration group-2 and -○- shows a compound administration group-3 respectively.

### Best Mode for Embodying the Invention

While the "compound having a gonadotropin releasing hormone (GnRH)-antagonistic action" (GnRH antagonist) may be any as long as it has a gonadotropin releasing hormone-antagonistic action, non-peptide compounds are preferable, and fused heterocyclic compounds are particularly preferable.

The fused heterocyclic compound is exemplified by the aforementioned compound (I), compound (VIII), salts thereof and the like.

Each substituent in the above-mentioned formula (I) is defined in the following.

The "C₁₋₄ alkoxy group" for R¹ or R² includes, for example, methoxy, ethoxy, propoxy, isopropoxy, butoxy, tert-butoxy and the like. Of these, preferred is a C₁₋₃ alkoxy group. More preferred is methoxy.

The "C₁₋₄ alkoxy-carbonyl group" for R¹ or R² includes, for example, methoxycarbonyl, ethoxycarbonyl, propoxycarbonyl, isopropoxycarbonyl, butoxycarbonyl, tert-butoxycarbonyl and the like. Of these, preferred is a C₁₋₃ alkoxy-carbonyl group. More preferred is methoxycarbonyl.

The "C₁₋₄ alkyl group" of the "C₁₋₄ alkyl group which may be substituted" for R¹ or R² includes, for example, a straight-chain C₁₋₄ alkyl group (e.g., methyl, ethyl, propyl, butyl, etc.), a branched C₃₋₄ alkyl group (e.g., isopropyl, isobutyl, sec-butyl, tert-butyl, etc.), and the like. Of these, preferred is a C₁₋₃ alkyl group. Particularly preferred is ethyl.

The "substituents" of the "C₁₋₄ alkyl group which may be substituted" for R¹ or R² include, for example, (i) hydroxy, (ii) C₁₋₇ acyloxy (e.g., C₁₋₆ alkyl-carbonyloxy such as acetoxy, propionyloxy, etc.), (iii) benzoyloxy, (iv) an amino group which may be substituted by 1 or 2 substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₄ acyl (e.g., C₁₋₃ alkyl-carbonyl such as acetyl, propionyl, etc.), C₁₋₄ alkyl (e.g., methyl, ethyl, propyl, butyl, etc.) and C₁₋₃ alkylsulfonyl (e.g., methanesulfonyl etc.), etc. [e.g., amino, dimethylamino, methoxycarbonylamino, ethoxycarbonylamino, tert-butoxycarbonylamino, benzyloxycarbonylamino, acetylamino, methanesulfonylamino, etc.], (v) C₁₋₁₀ alkoxy (e.g., methoxy, ethoxy, propoxy, tert-butoxy, etc.), (vi). C₃₋₇ cycloalkyloxycarbonyl-C₁₋₃ alkoxy (e.g., cyclohexyloxycarbonyloxy-1-ethoxy, etc.), (vii) C₁₋₃ alkoxy-C₁₋₃ alkoxy (e.g., methoxymethoxy, methoxyethoxy, etc.), and the like. Of these, preferred is hydroxy.

The "C₁₋₄ alkyl group" of the "C₁₋₄ alkyl group which may be substituted" for R¹ or R² may have 1 to 5, preferably 1 to 3, substituents as mentioned above at substitutable positions. When the number of substituents is two or more, those substituents may be the same as or different from each other.

Preferably, one of R¹ and R² is a hydrogen atom, and the other is a C₁₋₃ alkoxy group.

The "halogen atom" for R³ includes, for example, fluorine, chlorine, bromine and iodine. Of these, preferred is chlorine.

The "C₁₋₄ alkoxy group" of the "C₁₋₄ alkoxy group which may be substituted" for R³ includes, for example, methoxy, ethoxy, propoxy, isopropoxy, butoxy, tert-butoxy and the like. Of these, preferred is methoxy.

The "substituents" of the "C₁₋₄ alkoxy group which may be substituted" for R³ are the same as those mentioned above for the "substituents" of the "C₁₋₄ alkyl group which may be substituted" for R¹ or R². Of those, preferred is a C₁₋₄ alkoxy group.

The "C₁₋₄ alkoxy group" may have 1 to 5, preferably 1 to 3, substituent(s) as mentioned above at substitutable positions. When the number of substituents is two or more, those substituents may be the same as or different from each other.

The "C₁₋₄ alkylenedioxy group" formed by adjacent two R³ in combination includes, for example, methylenedioxy, ethylenedioxy and the like.

R³ is preferably a hydrogen atom.

The "C₁₋₄ alkyl group" for R⁴ includes, for example, a straight-chain C₁₋₄ alkyl group (e.g., methyl, ethyl, propyl, butyl, etc.), a branched C₃₋₄ alkyl group (e.g., isopropyl, isobutyl, sec-butyl, tert-butyl, etc.), and the like. Of these, preferred is a C₁₋₃ alkyl group. Particularly preferred is methyl.

The "C₁₋₄ alkyl group which may be substituted" for R⁶ includes, for example, "C₁₋₄ alkyl group which may be substituted" for R¹ or R².

The "heterocycle" formed by R⁴ and R⁵ in combination includes, for example, a 5- or 6-membered nitrogen-containing heterocyclic group. When R⁴ and R⁵ are bonded, examples of the group of the formula: 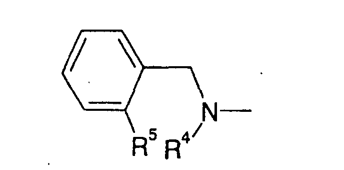 include a group of the formula: 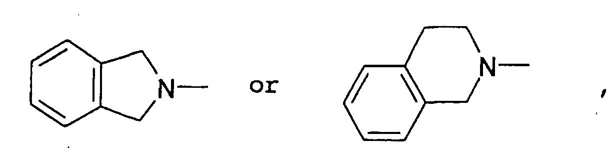 and the like.
Of these, preferred is a group of the formula:

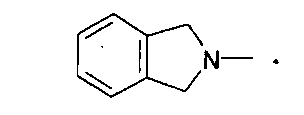

Preferably, R⁶ is a group of the formula: 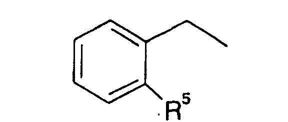 wherein R⁵ is as defined above.

Preferably, R⁴ is a C₁₋₃ alkyl group and R⁵ is a hydrogen atom.

Preferably, n is an integer of 0 to 2.

Preferable examples of compound (I) include a compound or a salt thereof, wherein R¹ is a hydroxy group, a methoxy group or a C₁₋₃ alkyl group; R² is a hydrogen atom or a C₁₋₃ alkyl group; R⁴ is a C₁₋₃ alkyl group; R⁶ is a benzyl group; and n is 0, and the like.

Of these, more preferred is a compound, wherein R¹ is a C₁₋₃ alkoxy group; R² and R⁵ are each a hydrogen atom; R⁴ is a C₁₋₃ alkyl group; R⁶ is a benzyl group; and n is 0, or a salt thereof, and the like.

As compound (I), concretely mentioned are 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-hydroxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-methylureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-ethylureido)phenyl]-3-phenylthieno[2,3-d]pyrimidine-2,4(1H,3H)-dione, and salts thereof.

Of these, preferred is 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof.

Each substituent in the above-mentioned formula (VIII) is defined in the following.

The "C₁₋₇ alkyl group" of the "C₁₋₇ alkyl group which may be substituted" for R⁹ includes, for example, a straight-chain C₁₋₇ alkyl group (e.g. methyl, ethyl, propyl, butyl, pentyl, hexyl, heptyl, etc.); a branched C₃₋₇ alkyl group (e.g., isopropyl, isobutyl, sec-butyl, tert-butyl, isopentyl, neopentyl, etc.) and the like. Of these, preferred is a branched C₃₋₇ alkyl group. Particularly preferred is isopropyl.

The "substituents" of the "C₁₋₇ alkyl group which may be substituted" for R⁹ include, for example, (i) a hydroxy group, (ii) C₁₋₇ acyloxy (e.g., C₁₋₆ alkyl-carbonyloxy such as acetoxy, propionyloxy, etc.; benzoyloxy etc.), (iii) amino which may be substituted by 1 or 2 substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl, propionyl, etc.), C₁₋₃ alkylsulfonyl (e.g., methanesulfonyl etc.) and C₁₋₃ alkyl (e.g., methyl, ethyl, etc.), and the like, which is exemplified by amino, methoxycarbonylamino, ethoxycarbonylamino, tert-butoxycarbonylbenzyloxycarbonylamino, acetylamino, methanesulfonylamino, methylamino, dimethylamino and the like, (iv) C₁₋₁₀ (preferably C₁₋₄) alkoxy which may be substituted by 1 to 3 substituent(s) selected from the group consisting of C₃₋₇ cycloalkyloxycarbonyl (e.g., cyclohexyloxycarbonyloxy, etc.) and C₁₋₃ alkoxy (e.g., methoxy, ethoxy, etc.), which is exemplified by methoxy, ethoxy, propoxy, tert-butoxy, cyclohexyloxycarbonyloxy-1-ethoxy, methoxymethoxy, ethoxymethoxy and the like, (v) C₁₋₆ alkoxycarbonyl (e.g., methoxycarbonyl, ethoxycarbonyl, propoxycarbonyl, etc.), and the like. Of these, preferred is a hydroxy group.

The "C₁₋₇ alkyl group" may have 1 to 5, preferably 1 to 3, substituent(s) as mentioned above at substitutable position(s). When the number of substituents is two or more, those substituents may be the same as or different from each other.

The "C₃₋₇ cycloalkyl group" of the "C₃₋₇ cycloalkyl group which may be substituted" for R⁹ includes, for example, cyclopropyl, cyclobutyl, cyclopentyl, cyclohexyl, cycloheptyl, and the like. Of these, preferred is cyclopropyl.

The "substituents" of the "C₃₋₇ cycloalkyl group which may be substituted" for R⁹ are the same as those mentioned above for the "substituents" of the "C₁₋₇ alkyl group which may be substituted" for R⁹. The number of substituents is 1 to 3. When the number of substituents is two or more, those substituents may be the same as or different from each other.

The "C₁₋₆ alkoxyamino group" of the "C₁₋₆ alkoxyamino group which may be substituted" for R⁹ includes, for example, a mono- or di-C₁₋₆ alkoxyamino group (e.g., methoxyamino, ethoxyamino, dimethoxyamino, diethoxyamino, ethoxymethoxyamino, etc.). Of these, preferred is a mono-C₁₋₃ alkoxyamino group (e.g., methoxyamino, etc.).

As the "substituents" of the "C₁₋₆ alkoxyamino group which may be substituted" for R⁹, for example, the same number of those similar to the "substituents" of the above-mentioned "C₁₋₇ alkyl group which may be substituted" for R⁹ can be mentioned. When the number of substituents is two or more, those substituents may be the same as or different from each other. The "C₁₋₆ alkoxy group" or the "nitrogen atom of an amino group" of the C₁₋₆ alkoxyamino group may be substituted by the above "substituents".

Such "C₁₋₆ alkoxyamino group which may be substituted" is exemplified by methoxyamino, N-methyl-N-methoxyamino, N-ethyl-N-methoxyamino, ethoxyamino, dimethoxyamino, diethoxyamino, ethoxymethoxyamino, and the like. Preferred is, for example, a C₁₋₃ alkoxyamino group, an N-C₁₋₃ alkyl-N-C₁₋₃ alkoxyamino group and the like.

The "substituents" of the "hydroxyamino group which may be substituted" for R⁹ may be substituted on the "hydroxy group" of the hydroxyamino group or the "nitrogen atom of an amino group" of the hydroxyamino group. Such "substituents" on the "hydroxy group" include, for example, (i) a C₁₋₇ acyloxy group (e.g., C₁₋₆ alkyl-carbonyloxy such as acetoxy, propionyloxy; benzoyloxy etc.), (ii) an amino group which may be substituted by 1 or 2 substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl, propionyl, etc.), C₁₋₃ alkylsulfonyl (e.g., methanesulfonyl etc.) and C₁₋₃ alkyl (e.g., methyl, ethyl, etc.) and the like, which is exemplified by amino, methoxycarbonylamino, ethoxycarbonylamino, tert-butoxycarbonylbenzyloxycarbonylamino, acetylamino, methanesulfonylamino, methylamino, dimethylamino and the like, (iii) a C₁₋₁₀ (preferably C₁₋₄) alkoxy group which may be substituted by 1 to 3 substituents selected from the group consisting of C₃₋₇ cycloalkyloxycarbonyl (e.g., cyclohexyloxycarbonyloxy, etc.) and C₁₋₃ alkoxy (e.g., methoxy, ethoxy, etc.), which is exemplified by methoxy, ethoxy, propoxy, tert-butoxy, cyclohexyloxycarbonyloxy-1-ethoxy, methoxymethoxy, ethoxymethoxy and the like, and the like. The "substituents" on the "nitrogen atom of the amino group" include, for example, the groups described in the above (i) to (iii) and a C₁₋₆ alkyl group (e.g., methyl, ethyl, propyl, isopropyl, butyl, isobutyl, sec-butyl, tert-butyl, pentyl, isopentyl, neopentyl, hexyl, etc.) and the like. The number of substituents is 1 to 5, preferably 1 to 3. When the number of substituents is two or more, those substituents may be the same as or different from each other.

Preferable examples of the "hydroxyamino group which may be substituted" include an N-C₁₋₆ alkyl-N-hydroxyamino group (e.g., N-methyl-N-hydroxyamino, N-ethyl-N-hydroxyamino, etc.) and the like. More preferred is an N-C₁₋₃ alkyl-N-hydroxyamino group and the like.

The "C₁₋₇ alkyl group" of the "C₁₋₇ alkyl group which may be substituted" for R¹⁰ includes, for example, a straight-chain or branched C₁₋₇ alkyl group (e.g., methyl, ethyl, propyl, isopropyl, butyl, isobutyl, sec-butyl, tert-butyl, pentyl, isopentyl, neopentyl, hexyl, heptyl, etc.) and the like. Of these, preferred is a C₁₋₃ alkyl group (e.g., methyl, ethyl, propyl, isopropyl, and the like. Particularly preferred is isopropyl.

As the "substituents" of the "C₁₋₇ alkyl group which may be substituted" for R¹⁰, for example, the same number of those similar to the "substituents" of the above-mentioned "C₁₋₇ alkyl group which may be substituted" for R⁹ can be mentioned. When the number of substituents is two or more, those substituents may be the same as or different from each other.

The "substituents" of the "phenyl group which may be substituted" for R¹⁰ includes, for example, halogen (e.g., fluorine, chlorine, bromine, iodine, etc.), a C₁₋₃ alkyl group (e.g., methyl, ethyl, propyl, isopropyl, etc.), and a C₁₋₃ alkoxy group (e.g., methoxy, ethoxy, propoxy, isopropoxy, etc.). Of these, preferred is halogen, more preferred is fluorine.

The "phenyl group" may have 1 to 5, preferably 1 to 3, substituents as mentioned above at substitutable positions and, when the number of substituents is two or more, those substituents may be the same as or different from each other.

R⁹ is preferably a substituted branched C₃₋₇ alkyl group or a substituted C₃₋₇ cycloalkyl group, more preferably a C₁₋₇ alkyl group substituted by a hydroxy group or a C₃₋₇ cycloalkyl group substituted by a hydroxy group. Of these, preferred is a substituted C₃₋₇ cycloalkyl group. Also, a C₁₋₃ alkyl group which may be substituted by a hydroxy group, a C₃₋₇ cycloalkyl group which may be substituted by a hydroxy group, mono-C₁₋₃ alkoxyamino, an N-C₁₋₃ alkyl-N-hydroxyamino group, a hydroxyamino group and the like are preferred. Especially preferable R⁹ is a cyclopropyl group which may be substituted by a hydroxy group or a methoxyamino group, and the like. Most preferred is a cyclopropyl group substituted by a hydroxy group.

R¹⁰ is preferably a C₁₋₇ alkyl group which may be substituted. More preferred is a C₁₋₃ alkyl group which may be substituted by a hydroxy group, and the like. Especially preferred is isopropyl. Phenyl is also preferred.

Preferable examples of compound (VIII) include a compound wherein R⁹ is a C₁₋₃ alkyl group which may be substituted by a hydroxy group, a C₃₋₇ cycloalkyl group which may be substituted by a hydroxy group or a mono-C₁₋₃ alkoxyamino group; and R¹⁰ is a C₁₋₃ alkyl group, or a salt thereof, and the like.

More preferred is a compound wherein R⁹ is (1) a C₁₋₄ alkyl group substituted by 1 or 2 hydroxy group(s), (2) a C₃₋ ₇ cycloalkyl group substituted by a hydroxy group, or (3) a C₁₋₃ alkoxyamino group; and
R¹⁰ is an isopropyl group or a phenyl group, or a salt thereof, and the like.

As compound (VIII), concretely mentioned are 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine,
5-benzoyl-3-(N-benzyl-N-methylaminomethyl)-7-(2,6-difluorobenzyl)-4,7-dihydro-4-oxo-2-[4-(3-hydroxy-2-methylpropionylamino)phenyl]thieno[2,3-b]pyridine,
5-(4-fluorobenzoyl)-3-(N-benzyl-N-methylaminomethyl)-7-(2,6-difluorobenzyl)-4,7-dihydro-4-oxo-2-(4-cyclopropanecarbonylaminophenyl)thieno[2,3-b]pyridine,
3-(N-benzyl-N-methylaminomethyl)-4,7-dihydro-5-isobutyryl-7-(2,6-difluorobenzyl)-2-[4-(3-hydroxy-2-methylpropionylamino)-phenyl]-4-oxothieno[2,3-b]pyridine,
3-(N-benzyl-N-methylaminomethyl)-4,7-dihydro-5-isobutyryl-7-(2,6-difluorobenzyl)-2-(4-N'-methoxyureidophenyl)-4-oxothieno[2,3-b]pyridine,
3-(N-benzyl-N-methylaminomethyl)-4,7-dihydro-5-isobutyryl-7-(2,6-difluorobenzyl)-2-[4-[(1-hydroxycyclopropyl)-carbonylamino]phenyl]-4-oxothieno[2,3-b]pyridine,
(R)-4,7-dihydro-2-[4-(3-hydroxy-2-methylpropionylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(2-hydroxy-2-methylpropionylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(3-hydroxy-3-methylbutyrylamino)phenyl]-7-(2,6-difluorobenzyl)-3-(N-benzyl-N-methylaminomethyl)-5-isobutyryl-4-oxothieno[2,3-b]pyridine,
(R)-4,7-dihydro-2-[4-(2,3-dihydroxypropionylamino)phenyl]-7-(2,6-difluorobenzyl)-3-(N-benzyl-N-methylaminomethyl)-5-isobutyryl-4-oxothieno[2,3-b]pyridine,
3-(N-benzyl-N-methylaminomethyl)-5-benzoyl-7-(2,6-difluorobenzyl)-4,7-dihydro-2-[4-[(1-hydroxycyclopropyl)-carbonylamino]phenyl]-4-oxothieno[2,3-b]pyridine, salts thereof and the like.

Of these, 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof is preferable.

Salts of compound (I) and (VIII) are preferably physiologically acceptable acid addition salts. Such salts include, for example, salts with inorganic acids (e.g., hydrochloric acid, hydrobromic acid, nitric acid, sulfuric acid, phosphoric acid, etc.), salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), and the like. When compound (I) has an acidic group, it may form a physiologically acceptable salt with an inorganic base (e.g., alkali metals and alkaline earth metals such as sodium, potassium, calcium and magnesium, ammonia etc.) or an organic base (e.g., trimethylamine, triethylamine, pyridine, picoline, ethanolamine, diethanolamine, triethanolamine, dicyclohexylamine, N,N'-dibenzylethylenediamine, etc).

Compound (I) can be produced, for example, in any per se known manner according to the methods disclosed in JP-A-9-169768, WO 96/24597 or analogous methods thereto. Concretely mentioned are the following Production method 1 and Production method 2. Compounds described in the reaction scheme may form a salt, which is exemplified by those recited for the salt of Compound (I).

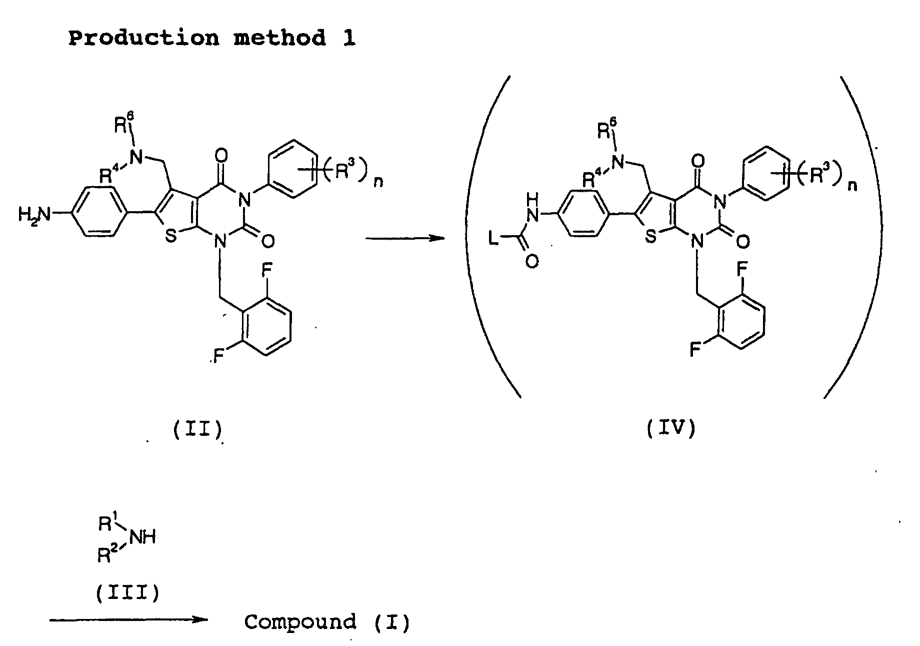

In the above formulae, L represents a leaving group, and other symbols are as defined above.

The "leaving group" for L includes, for example, 1-imidazolyl, a halogen atom, an alkoxy group which may be substituted, and the like. The "alkoxy group which may be substituted" includes, for example, a C₁₋₄ alkoxy group which may be substituted by 1 to 3 halogen atom(s) such as chlorine, bromine, etc. (e.g., a 2,2,2-trichloroethoxy group, etc.) and the like.

Compound (II) can be produced by the methods disclosed in JP-A-9-169768 or analogous methods thereto.

Compound (I) can be produced by reacting compound (II) with carbonyldiimidazole (N,N'-carbonyldiimidazole; CDI) or phosgene (inclusive of, dimer and trimer) and the like to obtain compound (IV), followed by a reaction with compound (III). The reaction can be carried out without isolation of compound (IV), or isolated and used in the next step.

Compound (IV) can be also produced by reacting compound (II) with a chloroformic acid ester compound (e.g., 2,2,2-trichloroethyl chloroformate, 1-chloroethyl chloroformate, etc.) and the like.

In the reaction of compound (II) with carbonyldiimidazole or phosgene, and the like, carbonyldiimidazole or phosgene, and the like is used in an amount of about 1 to 3 moles relative to 1 mole of compound (II).

This reaction is generally carried out in a suitable solvent inert to the reaction.

Examples of the solvent include ethers (e.g., ethyl ether, dioxane, dimethoxyethane, tetrahydrofuran, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and the like.

The reaction temperature is usually about 0 to about 150°C, preferably room temperature (about 15 to about 25°C). The reaction time is usually about 1 to about 36 hours.

This reaction is carried out in the presence of a base where necessary.

The "base" is exemplified by inorganic bases such as sodium carbonate, sodium hydrogencarbonate, potassium carbonate, potassium hydrogencarbonate, sodium hydroxide, potassium hydroxide and thallium hydroxide, and organic bases such as triethylamine and pyridine.

The amount of the "base" to be used is about 2 moles to 20 moles, preferably about 5 moles to 12 moles, relative to 1 mole of compound (II).

The following reaction with compound (III) can be carried out under the same conditions as those for the reaction of compound (II) with carbonyldiimidazole or phosgene. The amount of compound (III) to be used is about 2 to 20 moles, preferably about 5 to 10 moles, relative to 1 mole of compound (II) or compound (IV). The reaction temperature is usually about 0 to 150°C, preferably room temperature (about 15 to 25°C). The reaction time is usually about 1 to 6 hours.

Compound (III) and carbonyldiimidazole or phosgene may be reacted simultaneously with compound (II).

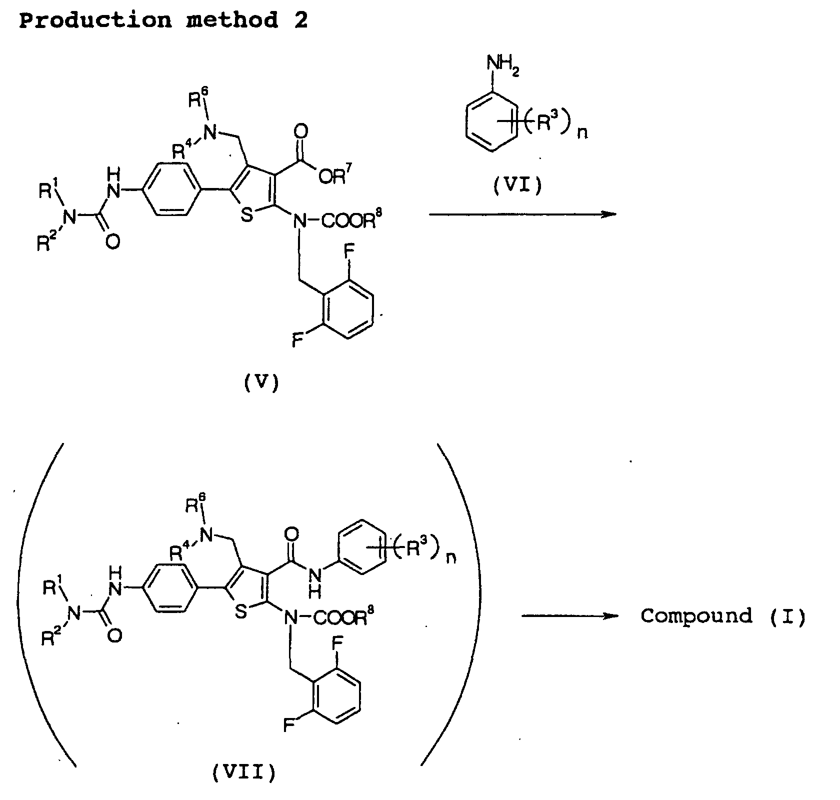

In the above formulae, R⁷ represents a hydrogen atom or an alkyl group, R⁸ represents an alkyl group, and other symbols are as defined above.

The "alkyl group" for R⁷ or R⁸ is exemplified by those similar to the "C₁₋₄ alkyl group" of the "C₁₋₄ alkyl group which may be substituted" for R¹ or R².

Compound (V) can be produced by a method known per se, such as a method comprising reacting p-nitrophenylacetone, a cyanoacetic acid ester derivative and sulphur (e.g., Chem. Ber., 99, 94-100(1966) etc.), and subjecting the obtained 2-amino-4-methyl-5-(4-nitrophenyl)thiophene to the methods disclosed in JP-A-9-169768, WO 96/24597 and the like, or methods analogous thereto.
1) When R⁷ is a hydrogen atom, compound (I) is produced by reacting compound (V) with a compound of the formula: 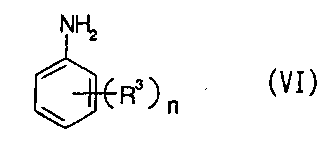 wherein each symbol is as defined above, or a salt thereof [hereinafter sometimes referred to briefly as compound (VI)], in the presence of a condensing agent, to obtain compound (VII), and subjecting the compound to cyclization.

The "condensing agent" includes, for example, benzotriazol-1-yloxytripyrrolidinophosphonium hexafluorophosphate (PyBOP) and the like.

The amount of the "condensing agent" to be used is about 1 to 3 moles relative to 1 mole of compound (V).

This reaction is generally carried out in a suitable solvent inert to the reaction.

Examples of the solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.) and the like.

The reaction temperature is usually about 0 to about 150°C, preferably room temperature (about 15 to about 25°C). The reaction time is usually about 1 to about 36 hours.

The product may be applied to the next reaction in the form of a reaction mixture or as a crude product. It is also possible to isolate the product from the reaction mixture according to a conventional method.

Compound (VII) is subjected to cyclization in the presence of a base.

The "base" is exemplified by inorganic bases such as sodium methoxide, sodium carbonate, sodium hydrogencarbonate, potassium carbonate, potassium hydrogencarbonate, sodium hydroxide, potassium hydroxide and thallium hydroxide, and organic bases such as triethylamine and pyridine.

The amount of the "base" to be used is about 2 moles to 20 moles, preferably about 5 moles to 12 moles, relative to 1 mole of compound (VII).

This reaction is generally carried out in a suitable solvent inert to the reaction.

Examples of the solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.) and the like.

The reaction temperature is usually about 0 to about 150°C, preferably room temperature (about 15 to about 25°C). The reaction time is usually about 1 to about 36 hours.
2) When R⁷ is an alkyl group, compound (I) is produced by reacting compound (V) with an activated compound (VI).

The activated compound (VI) can be produced according to a method known *per se* and obtained by, for example, reacting an organo-aluminum reagent with compound (VI) in a suitable solvent inert to the reaction.

The "organo-aluminum reagent" includes, for example, trimethyl aluminum, dimethyl aluminum chloride, and the like, a solution including these and the like.

The amount of the "organo-aluminum reagent" to be used is 1 to 5 moles, preferably 1 mole, relative to 1 mole of compound (VI).

Examples of the preferable solvent include halogenated hydrocarbons (e.g., chloroform, dichloromethane, etc.).

The reaction temperature is usually about 0 to 150°C, preferably room temperature (about 15 to 25°C). The reaction time is usually about 1 to 6 hours.

The cyclization can be carried out by reacting compound (V) with an activated compound (VI) to obtain compound (I).

The amount of the "compound (V)" to be used is preferably about 1/5 volume of a mixture of compound (VI) and the organo-aluminum reagent.

This reaction is generally carried out in a suitable solvent inert to the reaction.

Such solvent is preferable one used for the reaction to obtain an activated compound (VI).

The reaction temperature is usually about 0 to 150°C, preferably room temperature (about 15 to 25°C). The reaction time is usually about 1 to 48 hours.

Compound (I) may be isolated and purified by a separation method known *per se*, such as recrystallization, distillation chromatography, and the like.

When compound (I) is obtained in a free form, it can be converted to a objective salt by a method known *per* se or a method analogous thereto. When compound (I) is obtained in a salt form, it can be converted to a free form or an objective different salt by a method known *per* se or a method analogous thereto.

Compound (I) may be a hydrate or a non-hydrate. The hydrate is exemplified by monohydrate, sesquihydrate, dihydrate, and the like. When compound (I) is obtained as a mixture of optically active substances, it can be resolved into the objective (R)- and (S)-forms by the optical resolution techniques known *per se.*

Compound (I) may be labeled with an isotope (e.g., ³H, ¹⁴C, ³⁵S) and the like.

The compound (VIII) and a salt thereof can be produced by a method known *per se,* such as the method described in WO 95/28405, WO 00/00493 or a method analogous thereto.

Moreover, the fused heterocyclic compound to be used as the "compound having a gonadotropin releasing hormone (GnRH)-antagonistic action" is exemplified by the following compound (IX), a salt thereof, compounds described in USP 6159975, USP 6077858, USP 6077847, WO 00/53178, WO 00/53602, WO 00/53179, WO 00/53180, WO 00/53181, WO 00/53185, WO 00/69433, WO 99/51231, WO 99/51232, WO 99/51233, WO 99/51234, WO 99/51595, WO 99/51596, WO 95/28405, WO 97/14697, WO 97/14682, WO 96/24597, WO 00/69859 and the like, and the like.
[1] A compound of the formula (IX): 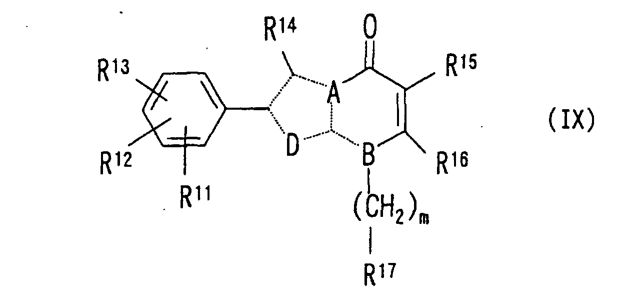 wherein one of A and D represents a nitrogen atom and the other represents a carbon atom, or both represent nitrogen atoms;
   B represents a nitrogen atom or a carbon atom;
   m represents an integer of 0 to 3;
   R¹¹, R¹² and R¹³ are the same or different and each represents (i) a hydrogen atom or (ii) a group bound via a carbon atom, a nitrogen atom, an oxygen atom or a sulfur atom;
   R¹⁴ represents a group bound via a carbon atom;
   R¹⁵ represents a hydrogen atom, halogen, or a group bound via a carbon atom or an oxygen atom;
   R¹⁶ represents a hydrogen atom or a group bound via a carbon atom;
   R¹⁷ represents a homocyclic group which may be substituted or a heterocyclic group which may be substituted; and
   dotted lines each represent a single bond or a double bond, or a salt thereof;
[2] a compound of the above [2] or a salt thereof, wherein R¹¹, R¹² and R¹³ are the same or different and each is
   (1) a hydrogen atom,
   (2) a hydrocarbon group which may be substituted,
   (3) an acyl group which may be substituted,
   (4) a heterocyclic group having a bond in a carbon atom thereof which may be substituted,
   (5) a group of the formula: -COO-R³¹ wherein R³¹ is a hydrogen atom, a hydrocarbon group which may be substituted or a heterocyclic group which may be substituted,
   (6) a group of the formula: -CO-NR²⁵R²⁶ wherein R²⁵ is a hydrogen atom, a hydrocarbon group which may be substituted or a C₁₋₁₀ alkoxy group, and R²⁶ is a hydrogen atom or a hydrocarbon group which may be substituted, or R²⁵ and R²⁶ form, taken together with the adjacent nitrogen atom, a cyclic amino group,
   (7) a cyano group,
   (8) a nitro group,
   (9) a group of the formula: -NR¹⁸R¹⁹ wherein R¹⁸ is (i) a hydrogen atom, (ii) a hydrocarbon group which may be substituted, (iii) an acyl group which may be substituted, (iv) a group of the formula: -O-R²³ wherein R²³ is a hydrogen atom, a C₁₋₁₀ hydrocarbon group which may be substituted, a C₁₋₂₀ acyl group which may be substituted, a C₁₋₂₀ alkylsulfonyl group which may be substituted, a C₆₋₁₄ arylsulfonyl group which may be substituted or a heterocyclic group which may be substituted, (v) a heterocyclic group which may be substituted or (vi) a group of the formula: -S(O)t-R²² wherein t is an integer of 0 to 2, and R²² is a hydrogen atom or a C₁₋₁₀ hydrocarbon group which may be substituted;
   R¹⁹ is a hydrogen atom, a hydrocarbon group which may be substituted or an acyl group which may be substituted; or
   R¹⁸ and R¹⁹ form, taken together with the adjacent nitrogen atom, a cyclic amino group which may be substituted,
   (10) a group of the formula: -O-R²³ wherein R²³ is as defined above, or
   (11) a group of the formula: -S(O)t-R²⁴ wherein t is an integer of 0 to 2, and R²⁴ is a hydrogen atom, a hydrocarbon group which may be substituted or a heterocyclic group which may be substituted;
      R¹⁴ is (1) a hydrocarbon group which may be substituted,

   (2) an acyl group which may be substituted,
   (3) a heterocyclic group having a bond in a carbon atom thereof which may be substituted,
   (4) a group of the formula: -COO-R³¹ wherein R³¹ is as defined above,
   (5) a group of the formula: -CO-NR²⁵R²⁶ wherein each symbol is as defined above, or
   (6) a cyano group;
   R¹⁵ is (1) a hydrogen atom,
   (2) halogen,
   (3) a hydrocarbon group which may be substituted,
   (4) an acyl group which may be substituted,
   (5) a heterocyclic group having a bond in a carbon atom thereof which may be substituted,
   (6) a group of the formula: -COO-R³¹ wherein R³¹ is as defined above,
   (7) a group of the formula: -CO-NR²⁵R²⁶ wherein each symbol is as defined above,
   (8) a cyano group, or
   (9) a group of the formula: -O-R²³ wherein R²³ is as defined above;
   R¹⁶ is (1) a hydrogen atom,
   (2) a hydrocarbon group which may be substituted,
   (3) an acyl group which may be substituted,
   (4) a heterocyclic group having a bond in a carbon atom thereof which may be substituted,
   (5) a group of the formula: -COO-R³¹ wherein R³¹ is as defined above,
   (6) a group of the formula: -CO-NR²⁵R²⁶ wherein each symbol is as defined above, or
   (7) a cyano group;
   R¹⁷ is (i) a C₆₋₁₀ aryl group or a C₃₋₇ cycloalkyl group, each of which may be substituted by 1 to 6 substituent(s) selected from the group consisting of (1) C₁₋₁₅ alkyl which may be substituted by 1 to 3 halogen(s), (2) C₃₋₁₀ cycloalkyl, (3) C₂₋₁₀ alkenyl,
   (4) C₂₋₁₀ alkynyl, (5) C₃₋₁₀ cycloalkenyl, (6) C₆₋₁₀ aryl, (7) C₇₋ ₂₀ aralkyl, (8) nitro, (9) hydroxy, (10) mercapto, (11) oxo, (12) thioxo, (13) cyano, (14) carbamoyl, (15) carboxyl, (16) C₁₋₆ alkoxy-carbonyl, (17) sulfo, (18) halogen, (19) C₁₋₆ alkoxy, (20) C₆₋₁₀ aryloxy, (21) C₁₋₆ alkanoyloxy, (22) C₁₋₆ alkylthio, (23) C₆₋₁₀ arylthio, (24) C₁₋₆ alkylsulfinyl, (25) C₆₋ ₁₀ arylsulfinyl, (26) C₁₋₆ alkylsulfonyl, (27) C₆₋₁₀ arylsulfonyl, (28) amino, (29) C₁₋₆ alkanoylamino, (30) mono- or di-C₁₋₄ alkylamino, (31) C₃₋₈ cycloalkylamino, (32) C₆₋₁₀ arylamino, (33) C₁₋₆ alkanoyl, (34) C₆₋₁₀ aryl-carbonyl and (35) 5- to 6-membered heterocyclic group, or
   (ii) a heterocyclic group which may be substituted,
   in which "hydrocarbon group" is a C₁₋₂₀ hydrocarbon group selected from a C₁₋₁₅ alkyl group, a C₃₋₁₀ cycloalkyl group, a C₂₋₁₀ alkenyl group, a C₂₋₁₀ alkynyl group, C₃₋₁₀ cycloalkenyl, a C₆₋₁₄ aryl group and a C₇₋₂₀ aralkyl group;
   "C₁₋₁₀ hydrocarbon group" is a C₁₋₁₀ alkyl group, a C₃₋₁₀ cycloalkyl group, a C₂₋₁₀ alkenyl group, a C₂₋₁₀ alkynyl group, C₃₋₁₀ cycloalkenyl, a C₆₋₁₀ aryl group or a phenyl-C₁₋₄ alkyl group;
   "acyl group" and "C₁₋₂₀ acyl group" are each formyl, C₁₋₆ alkyl-carbonyl, C₁₋₆ alkoxy-carbonyl, C₆₋₁₄ aryl-carbonyl, C₆₋₁₄ aryloxy-carbonyl, C₆₋₁₄ aryl-C₁₋₆ alkyl-carbonyl, C₆₋₁₄ aryl-C₁₋₆ alkoxy-carbonyl, C₂₋₄ alkenyl-carbonyl, C₃₋₆ cycloalkyl-carbonyl or tricyclic bridged C₉₋₁₀ hydrocarbon-carbonyl;
   "heterocyclic group" is (1) a 5- to 8-membered heterocyclic group containing 1 to 4 hetero atoms selected from oxygen atoms, sulfur atoms, nitrogen atoms and the like in addition to carbon atoms, (2) a bi- or tri-cyclic condensed heterocyclic group resulting from condensation of the same or different 2 or 3 of said heterocyclic groups, or (3) a bi- or tri-cyclic condensed heterocyclic group resulting from condensation of the above heterocyclic group and 1 or 2 benzene rings;
   "cyclic amino group" is a 5- to 7-membered nitrogenitrogen-containing cyclic group optionally containing 1 additional atom selected from oxygen atoms, sulfur atoms and nitrogen atoms;
   "substituent(s)" for the "hydrocarbon group which may be substituted", the "C₁₋₁₀ hydrocarbon group which may be substituted", the "acyl group which may be substituted", the "C₁₋₂₀ acyl group which may be substituted", the "C₁₋₂₀ alkylsulfonyl group which may be substituted" and the "'C₆₋₁₄ arylsulfonyl group which may be substituted" means 1 to 6 selected from (1) halogen, (2) nitro, (3) nitroso, (4) cyano, (5) hydroxy which may be substituted by (i) C₁₋₆ alkyl which may be substituted by 1 to 3 substituent(s) selected from the group consisting of hydroxy, C₁₋₆ alkoxy, C₁₋₃ alkoxy-C₁₋₃ alkoxy, C₁₋₃ alkylthio, hydroxy-C₁₋₃ alkoxy, C₁₋₆ alkyl-carbonyl, carboxyl, carbamoyl, C₁₋₆ alkyl-carbamoyl, a 5- to 8-membered heterocyclic group and halogen, (ii) C₁₋₄ alkanoyl or C₂₋₄ alkenoyl, (iii) C₆₋₁₄ aryl-C₁₋₆ alkyl which may be substituted by 1 to 3 substituent(s) selected from the group consisting of halogen, C₁₋₃ alkoxy and C₁₋₄ alkyl, (iv) C₆₋₁₄ aryl which may be substituted by 1 to 3 halogen(s), (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₂₋₆ alkenylamino, (x) C₁₋₃ alkoxy-carbonyl, (xi) formyl or C₁₋₆ alkyl-carbonyl, or (xii) C₃₋₆ cycloalkyloxy-carbonyl, (6) a group of the formula: -S(O)t-R²⁷ wherein t is an integer of 0 to 2, and R²⁷ is (i) a hydrogen atom or (ii) C₁₋ ₆ alkyl, C₆₋₁₄ aryl or C₇₋₂₀ aralkyl which may be substituted by 1 to 3 substituent(s) selected from the group consisting of halogen, nitro, cyano, hydroxy, oxo, thioxo, carboxyl, cyano-C₆₋₁₄ aryl and halogeno-C₆₋₁₄ aryl, (7) a group of the formula:-NR²⁸R²⁹ wherein R²⁸ and R²⁹ are the same or different and is a hydrogen atom, C₁₋₆ alkyl, C₁₋₆ alkylamino-C₁₋₆ alkyl, C₁₋₆ alkoxy, C₂₋₆ alkenyl, C₃₋₇ cycloalkyl, phenyl, phenyl-C₁₋₆ alkyl, C₁₋₆ alkanoyl, C₃₋₆ alkenoyl, C₄₋₇ cycloalkyl-carbonyl, phenyl-C₁₋ ₆ alkyl-carbonyl, C₁₋₆ alkoxy-carbonyl, phenyl-C₁₋₆ alkoxy-carbonyl or a 5- to 8-membered heterocyclic group, (8) a group of the formula: -CO-R³⁰ wherein R³⁰ is (i) a hydrogen atom, (ii) hydroxy, (iii) C₁₋₁₀ alkyl or (iv) C₁₋₆ alkoxy which may be substituted by C₆₋₁₄ aryl which may be substituted by 1 to 3 substituent(s) selected from the group consisting of halogen and nitro, (v) C₃₋₆ cycloalkyl, (vi) C₆₋₁₄ aryl, (vii) C₆₋₁₄ aryloxy, (viii) C₇₋₂₀ aralkyl, (ix) a group of the formula:-NR²⁰R²¹ wherein R²⁰ is a hydrogen atom, a C₁₋₁₀ hydrocarbon group which may be substituted, a C₁₋₂₀ acyl group which may be substituted, a group of the formula: -O-R²³ wherein R²³ is as defined above, a heterocyclic group which may be substituted or a group of the formula: -S(O)t-R²² wherein each symbol is as defined above; and R²¹ is a hydrogen atom or a C₁₋₁₀ hydrocarbon group; or R²⁰ and R²¹ form, taken together with the adjacent nitrogen atom, a cyclic amino group which may be substituted, or (x) a 5- to 8-membered heterocyclic group, (9)a 5- to 8-membered heterocyclic group which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (10) sulfo, (11) C₆₋₁₄ aryl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (12) C₃₋₇ cycloalkyl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (13) C₁₋₆ alkylenedioxy, (14) oxo, (15) thioxo, (16) C₂₋₄ alkynyl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (17) C₃₋ ₁₀ cycloalkyl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (18) C₂₋₁₀ alkenyl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (19) C₇₋₂₀ aralkyl which may be substituted by 1 to 3 substituent(s) selected from the group consisting of hydroxy, amino, mono- or di-C₁₋₄ alkylamino, C₁₋₄ alkoxy, halogen, nitro and C₁₋₆ alkyl, (20) amidino and (21) azido;
   "substituent(s)" for the "heterocyclic group which may be substituted" and the "heterocyclic group having a bond in a carbon atom thereof which may be substituted" means 1 to 6 selected from (1) C₁₋₆ alkyl, (2) C₂₋₆ alkenyl, (3) C₂₋₆ alkynyl, (4) C₃₋₆ cycloalkyl, (5) C₅₋₇ cycloalkenyl, (6) C₆₋₁₀ aryl-C₁₋₅ alkyl, (7) C₆₋₁₄ aryl, (8) C₁₋₆ alkoxy, (9) C₆₋₁₄ aryloxy, (10) C₁₋₆ alkanoyl, (11) C₆₋₁₄ aryl-carbonyl, (12) C₁₋₆ alkanoyloxy, (13) C₆₋₁₄ aryl-carbonyloxy, (14) carboxyl, (15) C₁₋₆ alkoxy-carbonyl, (16) carbamoyl, (17) N-mono-C₁₋₄ alkylcarbamoyl, (18) N,N-di-C₁₋₄ alkylcarbamoyl, (19) 3- to 6-membered cyclic aminocarbonyl, (20) halogen, (21) mono-, di- or tri-halogeno-C₁₋₄ alkyl, (22) oxo, (23) amidino, (24) imino, (25) amino, (26) mono- or di-C₁₋₄ alkylamino, (27) 3- to 6-membered cyclic amino, (28) C₁₋₆ alkanoylamino, (29) benzamido, (30) carbamoylamino, (31) N-C₁₋₄ alkylcarbamoylamino, (32) N,N-di-C₁₋ ₄ alkylcarbamoylamino, (33) C₁₋₃ alkylenedioxy, (34) -B(OH)₂, (35) hydroxy, (36) epoxy, (37) nitro, (38) cyano, (39) mercapto, (40) sulfo, (41) sulfino, (42) phosphono, (43) sulfamoyl, (44) C₁₋₆ alkylsulfamoyl, (45) di-C₁₋₆ alkylsulfamoyl, (46) C₁₋₆ alkylthio, (47) phenylthio, (48) C₁₋₆ alkylsulfinyl, (49) phenylsulfinyl, (50) C₁₋₆ alkylsulfonyl and (51) phenylsulfonyl; and
   "substituent(s)" for the "cyclic amino group which may be substituted" means 1 to 3 selected from C₁₋₆ alkyl, C₆₋₁₄ aryl, phenyl-C₁₋₄ alkyl, benzhydryl, C₁₋₆ alkyl-carbonyl, C₆₋₁₄ aryl-carbonyl and C₁₋₆ alkoxy-carbonyl;
[3] a compound of the above [1] or a salt thereof, wherein A is a nitrogen atom;
[4] a compound of the above [1] or a salt thereof, wherein B is a nitrogen atom;
[5] a compound of the above [1] or a salt thereof, wherein D is a nitrogen atom;
[6] a compound of the above [1] or a salt thereof, wherein m is 1;
[7] a compound of the above [1] or a salt thereof, wherein R¹¹ is a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a nitro group, a group of the formula: -NR²⁰R²¹ wherein R²⁰ is a hydrogen atom, a C₁₋₁₀ hydrocarbon group which may be substituted, a C₁₋₂₀ acyl group which may be substituted, a hydroxy group which may be substituted, a heterocyclic group which may be substituted or a group of the formula: -S(O)t-R²² wherein t is an integer of 0 to 2, and R²² is a hydrogen atom or a C₁₋₁₀ hydrocarbon group which may be substituted; R²¹ is a hydrogen atom or a C₁₋₁₀ hydrocarbon group; or R²⁰ and R²¹ form, taken together with the adjacent nitrogen atom, a cyclic amino group which may be substituted, or a group of the formula: -O-R²³ wherein R²³ is a hydrogen atom, a C₁₋₁₀ hydrocarbon group which may be substituted, a C₁₋₂₀ acyl group which may be substituted, a C₁₋₂₀ alkylsulfonyl group which may be substituted, a C₆₋₁₄ arylsulfonyl group which may be substituted, or a heterocyclic group which may be substituted; and R¹² and R¹³ are each a hydrogen atom;
[8] a compound of the above [1] or a salt thereof, wherein R¹² and R¹³ are each a hydrogen atom;
[9] a compound of the above [1] or a salt thereof, wherein R¹¹ is substituted at the para-position;
[10] a compound of the above [9] or a salt thereof, wherein R¹¹ is (1) an amino group which may be substituted by (i) carbamoyl which may be substituted by C₁₋₆ alkyl or C₁₋₆ alkoxy, or (ii) C₁₋₆ alkyl-carbonyl, or (2) a C₁₋₆ alkoxy group which may be substituted by C₃₋₆ cycloalkyl;
[11] a compound of the above [1] or a salt thereof, wherein R¹⁴ is a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted;
[12] a compound of the above [1] or a salt thereof, wherein R¹⁴ is a C₁₋₆ alkyl group.which may be substituted;
[13] a compound of the above [1] or a salt thereof, wherein R¹⁴ is a C₁₋₆ alkyl group which may be substituted by halogen, hydroxy which may be substituted or amino which may be substituted;
[14] a compound of the above [1] or a salt thereof, wherein R¹⁴ is a group of the formula: -(CH₂)n-NR²⁰R²¹ wherein n is an integer of 1 to 3; R²⁰ is a hydrogen atom, a C₁₋₁₀ hydrocarbon group which may be substituted, a C₁₋₂₀ acyl group which may be substituted, a hydroxy group which may be substituted, a heterocyclic group which may be substituted, or a group of the formula: -S(O)t-R²² wherein t is an integer of 0 to 2, and R²² is a hydrogen atom or a C₁₋₁₀ hydrocarbon group which may be substituted; and R²¹ is a hydrogen atom or a C₁₋₁₀ hydrocarbon group; or R²⁰ and R²¹ form, taken together with the adjacent nitrogen atom, a cyclic amino group which may be substituted;
[15] a compound of the above [1] or a salt thereof, wherein R¹⁴ is an N-C₁₋₆ alkyl-N-benzylaminomethyl group;
[16] a compound of the above [1] or a salt thereof, wherein R¹⁵ is a hydrogen atom, halogen, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a carboxyl group which may be esterified or amidated, or the formula: -O-R²³ wherein R²³ is a hydrogen atom or a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a C₁₋₂₀ alkylsulfonyl group which may be substituted, a C₆₋₁₄ arylsulfonyl group which may be substituted or a heterocyclic group which may be substituted;
[17] a compound of the above [1] or a salt thereof, wherein R¹⁵ is (1) a C₁₋₆ alkoxy-carbonyl group, (2) a C₆₋₁₄ aryl group which may be substituted by halogen or C₁₋₆ alkoxy, or (3) a phenyl-C₁₋₃ alkyl group;
[18] a compound of the above [1] or a salt thereof, wherein R¹⁶ is a hydrogen atom, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted;
[19] a compound of the above [1] or a salt thereof, wherein R¹⁶ is a hydrogen atom or a C₁₋₆ alkyl group;
[20] a compound of the above [1] or a salt thereof, wherein R¹⁷ is a C₆₋₁₄ aryl group which may be substituted;
[21] a compound of the above [1] or a salt thereof, wherein R¹⁷ is a phenyl group which may be substituted by halogen(s);
[22] a compound of the above [1] or a salt thereof, wherein one of A and D represents a nitrogen atom and the other represents a carbon atom, or both represent nitrogen atoms; B represents a nitrogen atom or a carbon atom; m represents an integer of 0 to 3; R¹¹, R¹² and R¹³ are the same or different and each represents (i) a hydrogen atom or (ii) a group bound via a carbon atom, a nitrogen atom, an oxygen atom or a sulfur atom; R¹⁴ represents a group bound via a carbon atom; R¹⁵ represents a hydrogen atom or a group bound via a carbon atom or an oxygen atom; R¹⁶ represents a hydrogen atom or a group bound via a carbon atom; R¹⁷ represents a homocyclic group which may be substituted or a heterocyclic group which may be substituted; and dotted lines each represent a single bond or a double bond;
[23] a compound of the above [1], which is represented by the formula (e): 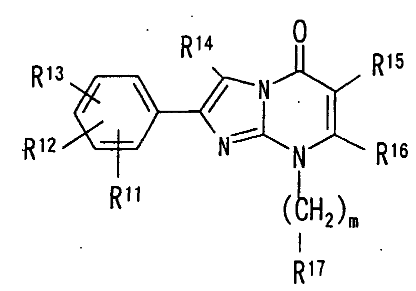 wherein each symbol is as defined above, or a salt thereof;
[24] a compound of the above [23] or a salt thereof, wherein R¹⁴ is a group of the formula: -(CH₂)n-NR²⁰R²¹ wherein n is an integer of 1 to 3; R²⁰ is a hydrogen atom, a C₁₋₁₀ hydrocarbon group which may be substituted, a C₁₋₂₀ acyl group which may be substituted, a hydroxy group which may be substituted, a heterocyclic group which may be substituted, or a group of the formula: -S(O)t-R²² wherein t is an integer of 0 to 2, and R²² is a hydrogen atom or a C₁₋₁₀ hydrocarbon group which may be substituted; and
   R²¹ is a hydrogen atom, a C₁₋₁₀ hydrocarbon group or a C₁₋₂₀ acyl group which may be substituted; or R²⁰ and R²¹ form, taken together with the adjacent nitrogen atom, a cyclic amino group which may be substituted;
[25] a compound of the above [1], which is represented by the formula: 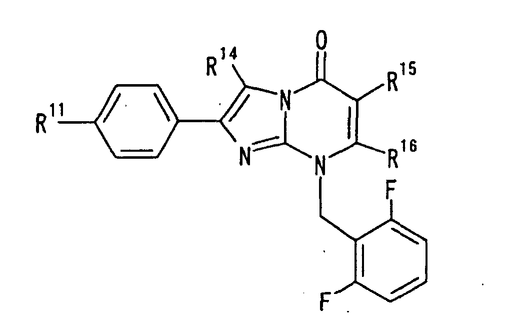 wherein each symbol is as defined above, or a salt thereof;
[26] a compound of the above [25] or a salt thereof, wherein R¹¹ is (1) an amino group which may be substituted by (i) carbamoyl which may be substituted by C₁₋₆ alkyl or C₁₋₆ alkoxy, or (ii) C₁₋₆ alkyl-carbonyl, or (2) a C₁₋₆ alkoxy group which may be substituted by C₃₋₆ cycloalkyl;
   R¹⁴ is an N-C₁₋₆ alkyl-N-benzylaminomethyl group;
   R¹⁵ is (1) a C₁₋₆ alkoxy-carbonyl group, (2) a C₆₋₁₀ aryl group which may be substituted by halogen or C₁₋₆ alkoxy, or (3) a phenyl-C₁₋₃ alkyl group; and
   R¹⁶ is a hydrogen atom;
[27] a compound of the above [25] or a salt thereof, wherein R¹¹ is (1) a nitro group,
   (2) an amino group which may be substituted by 1 or 2 substituent(s) selected from the group consisting of (i) C₁₋₆ alkyl which may be substituted by hydroxy, (ii) C₁₋₆ alkyl-carbonyl which may be substituted by hydroxy, halogen or thienyl, (iii) C₆₋₁₀ aryl-carbonyl which may be substituted by C₁₋₆ alkyl, C₁₋₆ alkoxy or halogen, (iv) C₃₋₆ cycloalkyl-carbonyl, (v) C₂₋₄ alkenyl-carbonyl, (vi) C₁₋₆ alkoxy-carbonyl, (vii) C₁₋₆ alkylamino-carbonyl, (viii) C₁₋₆ alkoxyaminocarbonyl, (ix) phenylaminocarbonyl, (x) isoxazolylcarbonyl, thienylcarbonyl, thiazolylcarbonyl, pyrazolylcarbonyl or furylcarbonyl, which may be substituted by 1 or 2 substituent(s) selected from the group consisting of C₁₋₆ alkyl, nitro and C₁₋₆ alkoxy, (xi) pyridylcarbonyl, (xii) C₁₋₆ alkylsulfonyl, (xiii) thienylsulfonyl and (xiv) phenylsulfonyl which may be substituted by C₁₋₆ alkyl,
   (3) a pyrrolyl group or
   (4) a hydroxy group which may be substituted by C₁₋₆ alkyl, C₃₋₆ cycloalkyl-C₁₋₃ alkyl or C₁₋₆ alkyl-carbonyl; R¹⁴ is a C₁₋₆ alkyl group which may be substituted by 1 or 2 substituent(s) selected from the group consisting of (1) halogen, (2) hydroxy and (3) amino which may be substituted by 1 or 2 substituent(s) selected from the group consisting of C₁₋ ₆ alkyl, phenyl-C₁₋₃ alkyl and di-C₁₋₆ alkylamino-C₁₋₃ alkyl; R¹⁵ is (1) halogen, (2) a phenyl group which may be substituted by halogen or C₁₋₆ alkyl, or (3) a carbonyl group substituted by (i) C₁₋₆ alkyl, (ii) amino substituted by C₁₋₆ alkyl and C₁₋₆ alkoxy or (iii) C₁₋₆ alkoxy; and
   R¹⁶ is a hydrogen atom or a C₁₋₃ alkyl group;
[28] 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid ethyl ester,
   8-(2,6-difluorobenzyl)-5,8-dihydro-2-[4-(methoxyaminocarbonylamino)phenyl]-3-(N-methyl-N-benzylaminomethyl)-5-oxoimidazo[1,2-a]pyrimidine-6-carboxylic acid isopropyl ester,
   8-(2,6-difluorobenzyl)-5,8-dihydro-2-[4-(ethylaminocarbonylamino)phenyl]]-3-(N-methyl-N-benzylaminomethyl)-5-oxoimidazo[1,2-a]pyrimidine-6-carboxylic acid isopropyl ester, or salts thereof.

In the above-mentioned formula (IX), each substituent is defined as follows.

In the above formulas, the group bound via a carbon atom includes, for example, (1) a hydrocarbon group which may be substituted, (2) an acyl group which may be substituted, (3) a heterocyclic group having a bond in a carbon atom thereof which may be substituted, (4) a carboxyl group which may be esterified or amidated, and (5) a cyano group.

In the above formulas, the group bound via a nitrogen atom includes, for example, (1) a nitro group or (2) a group of the formula: -NR¹⁸R¹⁹ wherein R¹⁸ represents hydrogen, a hydrocarbon group which may be substituted, an acyl group which may be substituted, a hydroxyl group which may be substituted, a heterocyclic group which may be substituted, or a group of the formula: -S(O)t-R²² wherein t represents an integer of 0 to 2, and R²² represents a hydrogen atom or a C₁₋₁₀ hydrocarbon group which may be substituted; R¹⁹ represents hydrogen, a hydrocarbon group which may be substituted or an acyl group which may be substituted; or R¹⁸ and R¹⁹ may form, taken together with the adjacent nitrogen atom, a cyclic amino group which may be substituted.

In the above formulas, the group bound via an oxygen atom includes, for example, a hydroxyl group which may be substituted. The hydroxyl group which may be substituted is represented by the formula: -O-R²³ wherein R²³ represents a hydrogen atom or a C₁₋₁₀ hydrocarbon group which may be substituted, a C₁₋₂₀ acyl group which may be substituted, a C₁₋₂₀ alkylsulfonyl group which may be substituted, a C₆₋₁₄ arylsulfonyl group which may be substituted or a heterocyclic group which may be substituted.

In the above formulas, the group bound via a sulfur atom includes, for example, a group of the formula: -S(O)t-R²⁴ wherein t represents an integer of 0 to 2, and R²⁴ represents a hydrogen atom or a hydrocarbon group which may be substituted or a heterocyclic group which may be substituted.

The above-described carboxyl group which may be esterified includes, for example, a group of the formula: -COO-R³¹ wherein R³¹ represents a hydrogen atom or a C₁₋₁₀ hydrocarbon group which may be substituted.

The above-described carboxyl group which may be amidated includes, for example, a group of the formula: -CO-NR²⁵R²⁶ wherein R²⁵ represents a hydrogen atom, a hydrocarbon group which may be substituted or an alkoxy group; R²⁶ represents a hydrogen atom or a hydrocarbon group which may be substituted; or R²⁵ and R²⁶ may form, taken together with the adjacent nitrogen atom, a cyclic amino group which may be substituted. The carboxyl group which may be amidated is preferably exemplified by a group represented by -CONH₂, and mono- or di-C₁₋₁₅ alkylcarbamoyl groups, preferably mono- or di-C₁₋₁₀ alkylcarbamoyl groups (e.g., methylcarbamoyl, ethylcarbamoyl, hexylcarbamoyl, dimethylcarbamoyl, methylethylcarbamoyl, etc.) and the like.

The hydrocarbon group in the above-described hydrocarbon group which may be substituted is preferably, for example, a C₁₋₂₀ hydrocarbon group, preferably a C₁₋₁₀ hydrocarbon group. The C₁₋₂₀ hydrocarbon group is exemplified by (1) a C₁₋₁₅ alkyl group (e.g., methyl, ethyl, n-propyl, isopropyl, n-butyl, sec-butyl, t-butyl, pentyl, hexyl, heptyl, octyl, nonyl, decyl, undecyl, dodecyl, tridecyl, tetradecyl, pentadecyl and the like; preferably C₁₋₁₀ alkyl, particularly preferably a C₁₋₆ alkyl group), (2) a C₃₋₁₀ cycloalkyl group (e.g., cyclopropyl, cyclobutyl, cyclopentyl, cyclohexyl, cycloheptyl, cyclooctyl, cyclononyl and the like; preferably a C₃₋₆ cycloalkyl group), (3) a C₂₋₁₀ alkenyl group (e.g., vinyl, allyl, isopropenyl, 1-butenyl, 2-butenyl, 3-butenyl, butadienyl, 2-methylallyl, hexatrienyl, 3-octenyl, etc.; preferably a C₂₋₆ alkenyl group), (4) a C₂₋₁₀ alkynyl group (e.g., ethynyl, 2-propynyl, isopropynyl, butynyl, t-butynyl, 3-hexynyl, etc.; preferably a C₂₋₆ alkynyl group), (5) a C₃₋₁₀ cycloalkenyl (e.g., cyclopropenyl, cyclopentenyl, cyclohexenyl, etc.; preferably a C₃₋₆ cycloalkenyl group), (6) a C₆₋₁₄ aryl group (e.g., phenyl, naphthyl, anthryl, phenanthryl, acenaphthyl, anthracenyl, etc.; preferably phenyl and naphthyl), (7) a C₇₋ ₂₀ aralkyl group (e.g., a C₆₋₁₄ aryl-C₁₋₆ alkyl group such as benzyl, phenethyl and benzhydryl, preferably a phenyl-C₁₋₆ alkyl group such as benzyl and phenethyl), and the like.

Such hydrocarbon groups may have 1 to 6, preferably 1 to 5, and more preferably 1 to 3, substituent(s) at any substitutable positions. Such substituents include, for example, (1) halogen, (2) nitro, (3) nitroso, (4) cyano, (5) hydroxyl which may be substituted, such as hydroxy which may be substituted by (i) C₁₋₆ alkyl [the C₁₋₆ alkyl may be substituted by 1 to 3 substituent(s) such as hydroxy, C₁₋₆ alkoxy, C₁₋₃ alkoxy-C₁₋₃ alkoxy, C₁₋₃ alkylthio, hydroxy-C₁₋₃ alkoxy, C₁₋₆ alkyl-carbonyl, carboxy, carbamoyl, C₁₋₆ alkyl-carbamoyl, a 5- to 8-membered heterocyclic group (same as the "5- to 8-membered heterocyclic group containing 1 to 4 hetero atom(s) selected from oxygen atoms, sulfur atoms, nitrogen atoms etc., in addition to carbon atoms" described below) and halogen], (ii) C₁₋₄ acyl (C₁₋₄ alkanoyl, C₂₋₄ alkenoyl, etc.), (iii) C₇₋₂₀ aralkyl (the C₇₋₂₀ aralkyl group is C₆₋₁₄ aryl-C₁₋₆ alkyl and may be substituted by 1 to 3, preferably 1, halogen, C₁₋₃ alkoxy or C₁₋₄ alkyl), (iv) C₆₋₁₄ aryl (the C₆₋₁₄ aryl may be substituted by 1 to 3, preferably 1, halogen), (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₂₋₆ alkenylamino, (x) C₁₋₃ alkoxy-carbonyl, (xi) C₁₋₆ alkyl-carbonyl or (xii) C₃₋₆ cycloalkyloxy-carbonyl, (6) a group of the formula: -S(O)t-R²⁷ wherein t represents an integer of 0 to 2; R²⁷ represents a hydrogen atom or a hydrocarbon group which may be substituted by 1 to 3, preferably 1, substituent (e.g., halogen, nitro, cyano, hydroxy, oxo, thioxo, carboxy, cyano-C₆₋₁₄ aryl, halogeno-C₆₋₁₄ aryl, etc.) at any substitutable positions; the hydrocarbon group is preferably a C₁₋₂₀ hydrocarbon group, particularly C₁₋₆ alkyl, C₆₋₁₄ aryl or C₇₋₂₀ aralkyl, (7) an amino group which may be substituted, such as a group of the formula: -NR²⁸R²⁹ wherein R²⁸ and R²⁹ are the same or different and each represents a hydrogen atom, C₁₋₆ alkyl, C₁₋₆ alkylamino-C₁₋₆ alkyl, C₁₋₆ alkoxy, C₂₋₆ alkenyl, C₃₋₇ cycloalkyl, phenyl, phenyl-C₁₋₆ alkyl, C₁₋₆ alkanoyl, C₃₋₆ alkenoyl, C₄₋₇ cycloalkyl-carbonyl, phenyl-C₁₋₆ alkyl-carbonyl, C₁₋₆ alkoxy-carbonyl, phenyl-C₁₋₆ alkoxy-carbonyl, or a 5- to 8-membered heterocyclic group (same as the "5- to 8-membered heterocyclic group containing 1 to 4 hetero atoms selected from oxygen atoms, sulfur atoms, nitrogen atoms etc., in addition to carbon atoms" described below) (8) a group of the formula: -CO-R³⁰ wherein R³⁰ represents (i) a hydrogen atom, (ii) hydroxyl, (iii) C₁₋₁₀ alkyl, (iv) C₁₋₆ alkoxy (this alkoxy may be substituted by C₆₋ ₁₄ aryl which may be substituted by 1 to 3, preferably 1, substituent such as halogen and nitro, at any substitutable positions), (v) C₃₋₆ cycloalkyl, (vi) C₆₋₁₄ aryl, (vii) C₆₋₁₄ aryloxy, (viii) C₇₋₂₀ aralkyl, (ix) an amino group which may be substituted and represented by the formula: -NR²⁰R²¹ wherein R²⁰ and R²¹ are as defined above, or (x) a 5- to 8-membered heterocyclic group (same as the "5- to 8-membered heterocyclic group containing 1 to 4 hetero atoms selected from oxygen atoms, sulfur atoms, nitrogen atoms etc., in addition to carbon atoms" described below) (e.g., C₁₋₆ alkanoyl, C₃₋₆ alkenoyl, C₁₋₆ alkoxy-carbonyl and the like are preferred), (9) a 5- to 8-membered heterocyclic group containing 1 to 4 hetero atoms selected from nitrogen atoms, oxygen atoms and sulfur atoms, (10) sulfo, (11) C₆₋₁₄ aryl, (12) C₃₋₇ cycloalkyl, (13) C₁₋₆ alkylenedioxy (e.g., methylenedioxy, ethylenedioxy, propylenedioxy, 2,2-dimethylenedioxy, etc.), (14) oxo, (15) thioxo, (16) C₂₋₄ alkynyl, (17) C₃₋₁₀ cycloalkyl, (18) C₂₋₁₀ alkenyl (preferably a C₂₋₆ alkenyl group), (19) C₇₋₂₀ aralkyl (e.g., C₆₋₁₄ aryl-C₁₋₆ alkyl), (20) amidino, and (21) azide, and the like.

Of the above-mentioned substituents on the hydrocarbon groups having substituents, (9) a 5- to 8-membered heterocyclic group containing 1 to 4 hetero atom(s) selected from nitrogen atoms, oxygen atoms and sulfur atoms, (11) C₆₋ ₁₄ aryl, (12) C₃₋₇ cycloalkyl, (16) C₂₋₄ alkynyl, (17) a C₃₋₁₀ cycloalkyl group, (18) a C₂₋₁₀ alkenyl group and (19) C₇₋₂₀ aralkyl and the like may further have 1 to 4, preferably 1 to 3, substituent(s) at any substitutable positions. Such substituents which may be further contained include, for example, 1 to 3, more preferably 1 or 2, group(s) selected from the group consisting of (1) hydroxyl, (2) amino, (3) mono- or di-C₁₋₄ alkylamino (e.g., methylamino, ethylamino, propylamino, dimethylamino, diethylamino, etc.), (4) C₁₋₄ alkoxy, (5) halogen, (6) nitro, (7) C₁₋₆ alkyl and the like.

When the hydrocarbon group is, for example, a cycloalkyl, cycloalkenyl, aryl or aralkyl group, it may be substituted by 1 to 3 C₁₋₆ alkyl(s). The C₁₋₆ alkyl may be further substituted by 1 to 3 substituent(s) such as hydroxy, oxo, C₁₋₃ alkoxy, C₁₋₃ alkylthio, halogen, carbamoyl and the like.

Such substituted C₁₋₆ alkyl is exemplified by formyl (resulting from methyl substitution by oxo), carboxyl (resulting from methyl substitution by oxo and hydroxy), C₁₋₆ alkoxycarbonyl (resulting from methyl substitution by oxo and alkoxy) (e.g., C₁₋₆ alkoxycarbonyl such as methoxycarbonyl, ethoxycarbonyl and t-butoxycarbonyl), hydroxy-C₁₋₆ alkyl (e.g., hydroxymethyl, hydroxyethyl, hydroxybutyl, hydroxypropyl, etc.), and C₁₋₃ alkoxy-C₁₋₆ alkyl (e.g., methoxymethyl, ethoxymethyl, ethoxybutyl, propoxymethyl, propoxyhexyl, etc.), and the like.

Although the number of the above-mentioned substituents ranges from 1 to 6, it is preferably 1 to 5, particularly preferably 1 to 3, and most preferably 1 or 2. The number of substituents that the substituents may have is preferably 1 to 4, particularly preferably 1 to 3, and most preferably 1 or 2.

The acyl group of the above-described acyl group which may be substituted, which has been recited as examples of the group bound via a carbon atom, R¹⁸ and R¹⁹, includes, for example, a C₁₋₂₀ acyl group such as formyl, C₁₋₆ alkyl-carbonyl (e.g., acetyl, ethylcarbonyl, propylcarbonyl, tert-propylcarbonyl, etc.), C₁₋₆ alkoxy-carbonyl (e.g., methoxycarbonyl, ethoxycarbonyl, tert-butoxycarbonyl, etc.), C₆₋₁₄ aryl-carbonyl (e.g., benzoyl, naphthoyl, etc.), C₆₋₁₄ aryloxy-carbonyl (e.g., phenoxycarbonyl, etc.), C₇₋₁₅ aralkyl-carbonyl (e.g., C₆₋₁₄ aryl-C₁₋₆ alkyl-carbonyl such as benzylcarbonyl), C₇₋₁₉ aralkyloxy-carbonyl (e.g., C₆₋₁₄ aryl-C₁₋₆ alkoxy-carbonyl such as benzyloxycarbonyl), C₂₋₄ alkenyl-carbonyl (e.g., 2-propenylcarbonyl, etc.), C₃₋₆ cycloalkyl-carbonyl (e.g., cyclopropylcarbonyl, etc.) tricyclic C₉₋₁₀ bridged hydrocarbon-carbonyl (e.g., adamantylcarbonyl, etc.) and the like.

Substituents of the acyl group which may be substituted are exemplified by the same substituents as those recited as examples of the substituents of the above-described hydrocarbon group which may be substituted.

In the above formulas, the heterocyclic group or the heterocyclic group in the heterocyclic group which may be substituted includes, for example, 5- to 8-membered heterocyclic groups containing 1 to 4 hetero atom(s) selected from oxygen atoms, sulfur atoms, nitrogen atoms etc., in addition to carbon atoms, bicyclic or tricyclic condensed heterocyclic groups resulting from condensation of the same or different 2 or 3 of such heterocyclic groups, and bicyclic or tricyclic condensed heterocyclic groups resulting from condensation of such a heterocyclic group, 1 or 2 benzene rings, and the like.

Examples of the heterocyclic group include (1) 5-membered 'heterocyclic groups containing 1 to 4 hetero atom(s) selected from oxygen atoms, sulfur atoms, nitrogen atoms etc., in addition to carbon atoms, such as thienyl, furyl, pyrrolyl, pyrrolinyl, oxazolyl, thiazolyl, pyrazolyl, imidazolyl, imidazolinyl, isoxazolyl, isothiazolyl, 1,2,4-oxadiazolyl, 1,3,4-oxadiazolyl, furazanyl, 1,2,4-thiadiazolyl, 1,2,3-thiadiazolyl, 1,2,5-thiadiazolyl, 1,2,3-triazolyl, 1,2,4-triazolyl, triazinyl, triazolidinyl, and 1H- or 2H-tetrazolyl; and (2) 6-membered heterocyclic groups containing 1 to 4 hetero.atom(s) selected from oxygen atoms, sulfur atoms, nitrogen atoms etc., in addition to carbon atoms, such as pyridyl, pyrimidinyl, thiomorpholinyl, morpholinyl, triazinyl, pyrrolidinyl, piperidinyl, pyranyl, thiopyranyl, 1,4-oxazinyl, 1,4-thiazinyl, 1,3-thiazinyl, piperazinyl, triazinyl, oxotriazinyl, pyridazinyl and pyrazinyl, and the like. (3) Bicyclic or tricyclic condensed heterocyclic groups include bicyclic or tricyclic condensed heterocyclic groups containing 1 to 4 hetero atom(s) selected from oxygen atoms, sulfur atoms, nitrogen atoms etc., in addition to carbon atoms, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolizinyl, indolyl, quinolizinyl, 1,8-naphthylidinyl, purinyl, pteridinyl, dibenzofuranyl, carbazolyl, acridinyl, phenanthridinyl, chromanyl, benzoxazinyl, phenazinyl, phenothiazinyl, phenoxazinyl, and the like.

Examples of the substituents of the heterocyclic group which may be substituted include (1) C₁₋₆ alkyl, (2) C₂₋₆ alkenyl, (3) C₂₋₆ alkynyl, (4) C₃₋₆ cycloalkyl, (5) C₅₋₇ cycloalkenyl, (6) C₇₋₁₁ aralkyl (C₆₋₁₀ aryl-C₁₋₅ alkyl such as benzyl and phenethyl, preferably benzyl), (7) C₆₋₁₄ aryl (phenyl, naphthyl, anthryl, phenanthryl, acenaphtyl, anthracenyl, etc., preferably phenyl), (8) C₁₋₆ alkoxy, (9) C₆₋₁₄ aryloxy (e.g., phenoxy, etc.), (10) C₁₋₆ alkanoyl (e.g., formyl, acetyl, propionyl, n-butyryl, iso-butyryl, etc.), (11) C₆₋₁₄ arylcarbonyl (e.g., benzoyl, etc.), (12) C₁₋₆ alkanoyloxy (e.g., formyloxy, acetyloxy, propionyloxy, n-butyryloxy, iso-butyryloxy, etc.), (13) C₆₋₁₄ aryl-carbonyloxy (e.g., benzoyloxy, etc.), (14) carboxyl, (15) C₁₋ ₆ alkoxy-carbonyl (e.g., methoxycarbonyl, ethoxycarbonyl, n-propoxycarbonyl, iso-propoxycarbonyl, n-butoxycarbonyl, isobutoxycarbonyl, tert-butoxycarbonyl, etc.), (16) carbamoyl, (17) N-mono-C₁₋₄ alkylcarbamoyl (e.g., N-methylcarbamoyl, N-ethylcarbamoyl, N-propylcarbamoyl, N-isopropylcarbamoyl, N-butylcarbamoyl, etc.), (18) N,N-di-C₁₋₄ alkylcarbamoyl (e.g., N,N-dimethylcarbamoyl, N,N-diethylcarbamoyl, N,N-dipropylcarbamoyl, N,N-dibutylcarbamoyl, etc.), (19) 3- to 6-membered cyclic aminocarbonyl (e.g., 1-aziridinylcarbonyl, 1-azetidinylcarbonyl, 1-pyrrolidinylcarbonyl, 1-piperidinylcarbonyl, N-methylpiperazinylcarbonyl, morpholinocarbonyl, etc.), (20) halogen, (21) mono-, di- or tri-halogeno-C₁₋₄ alkyl (e.g., chloromethyl, dichloromethyl, trifluoromethyl, trifluoroethyl, etc.), (22) oxo, (23) amidino, (24) imino, (25) amino, (26) mono- or di-C₁₋₄ alkylamino (e.g., methylamino, ethylamino, propylamino, isopropylamino, butylamino, dimethylamino, diethylamino, dipropylamino, diisopropylamino, dibutylamino, etc.), (27) a 3- to 6-membered cyclic amino group which may contain 1 to 3 hetero atoms selected from oxygen atoms, sulfur atoms, nitrogen atoms etc., in addition to carbon atoms and a nitrogen atom (e.g., aziridinyl, azetidinyl, pyrrolidinyl, pyrrolinyl, pyrrolyl, imidazolyl, pyrazolyl, imidazolidinyl, piperidino, morpholino, dihydropyridyl, N-methylpiperazinyl, N-ethylpiperazinyl, etc.), (28) C₁₋₆ alkanoylamino (e.g., formamido, acetamido, trifluoroacetamido, propionylamido, butyrylamido, isobutyrylamido, etc.), (29) benzamido, (30) carbamoylamino, (31) N-C₁₋₄ alkylcarbamoylamino (e.g., N-methylcarbamoylamino, N-ethylcarbamoylamino, N-propylcarbamoylamino, N-isopropylcarbamoylamino, N-butylcarbamoylamino, etc.), (32) N,N-di-C₁₋₄ alkylcarbamoylamino (e.g., N,N-dimethylcarbamoylamino, N,N-diethylcarbamoylamino, N,N-dipropylcarbamoylamino, N;N-dibutylcarbamoylamino, etc.), (33) C₁₋₃ alkylenedioxy (e.g., methylenedioxy, ethylenedioxy, etc.), (34) -B(OH)₂, (35) hydroxyl, (36) epoxy (-O-), (37) nitro, (38) cyano, (39) mercapto, (40) sulfo, (41) sulfino, (42) phosphono, (43) sulfamoyl, (44) C₁₋₆ alkylsulfamoyl (e.g., N-methylsulfamoyl, N-ethylsulfamoyl, N-propylsulfamoyl, N-isopropylsulfamoyl, N-butylsulfamoyl, etc.), (45) di-C₁₋₆ alkylsulfamoyl (e.g., N,N-dimethylsulfamoyl, N,N-diethylsulfamoyl, N,N-dipropylsulfamoyl, N,N-dibutylsulfamoyl, etc.), (46) C₁₋₆ alkylthio (e.g., methylthio, ethylthio, propylthio, isopropylthio, n-butylthio, sec-butylthio, tert-butylthio, etc.), (47) phenylthio, (48) C₁₋₆ alkylsulfinyl (e.g., methylsulfinyl, ethylsulfinyl, propylsulfinyl, butylsulfinyl, etc.), (49) phenylsulfinyl, (50) C₁₋₆ alkylsulfonyl (e.g., methylsulfonyl, ethylsulfonyl, propylsulfonyl, butylsulfonyl, etc.), and (51) phenylsulfonyl and the like.

The number of substituents of the heterocyclic group which may be substituted is 1 to 6, preferably 1 to 3, and more preferably 1 or 2.

The heterocyclic group in the heterocyclic group having a bond in a carbon atom thereof which may be substituted is exemplified by 5- to 8-membered heterocyclic groups containing 1 to 4 hetero atom(s) selected from oxygen atoms, sulfur atoms, nitrogen atoms etc., in addition to carbon atoms, bicyclic or tricyclic condensed heterocyclic groups resulting from condensation of the same or different 2 or 3 of such heterocyclic groups, and bicyclic or tricyclic condensed heterocyclic groups resulting from condensation of such a heterocyclic group and 1 or 2 benzene rings, etc., which heterocyclic groups having a bond in a constituent carbon atom thereof.

Examples of the heterocyclic group having a bond in a carbon atom thereof include, for example, (1) 5-membered heterocyclic groups containing 1 to 4 hetero atoms selected from oxygen atoms, sulfur atoms, nitrogen atom(s) etc., in addition to carbon atoms, such as thienyl (e.g., 2- or 3-thienyl), furyl (e.g., 2- or 3-furyl), pyrrolyl (e.g., 2- or 3-pyrrolyl), oxazolyl (e.g., 2-, 4- or 5-oxazolyl), thiazolyl (e.g., 2-, 4- or 5-thiazolyl), pyrazolyl (e.g., 3-, 4- or 5-pyrazolyl), pyrrolidinyl (e.g., 2- or 3-pyrrolidinyl), imidazolyl (e.g., 2-, 4- or 5-imidazolyl), imidazolinyl (e.g., 2-imidazolinyl, 2-imidazolidinyl), isoxazolyl (e.g., 3-, 4- or 5-isoxazolyl), isothiazolyl (e.g., 3-, 4- or 5-isothiazolyl), oxadiazolyl [e.g., 3- or 5-(1,2,4-oxadiazolyl), 2-, 5- or 6-(1,3,4-oxadiazolyl)], thiadiazolyl [e.g., 3- or 5-(1,2,4-thiadiazolyl, 2- or 5-(1,3,4-thiadiazolyl), 4- or 5-(1,2,3-thiadiazolyl), 3- or 4-(1,2,5-thiadiazolyl)], and triazolyl [e.g., 2- or 5-(1,2,3-triazolyl), 3- or 5-(1,2,4-triazolyl)], tetrazolyl [e.g., 5-(1H- or 2H-tetrazolyl)]; (2) 6-membered heterocyclic groups containing 1 to 4 hetero atom(s) selected from oxygen atoms, sulfur atoms, nitrogen atoms etc., in addition to carbon atoms, such as pyridyl (e.g., 2-, 3- or 4-pyridyl), pyrimidinyl (e.g., 2-, 4- or 5-pyrimidinyl), thiomorpholinyl (e.g., 2- or 3-thiomorpholinyl), morpholinyl (e.g., 2- or 3-morpholinyl), triazinyl (e.g., 3- or 6-triazinyl), piperidinyl (e.g., 2-, 3- or 4-piperidinyl), pyranyl (e.g., 2- or 3-pyranyl), thiopyranyl (e.g., 2- or 3-thiopyranyl), oxazinyl [e.g., 2-or 3-(1,4-oxazinyl)], thiazinyl [e.g., 2-or 3-(1,4-thiazinyl), 1- or 4-(1,3-thiazinyl)], piperazinyl (e.g., 2- or 3-piperazinyl), triazinyl (e.g., 3- or 6-triazinyl), pyridazinyl (e.g., 3- or 4-pyridazinyl) pyrazinyl (e.g., 2- or 3-pyrazinyl), pyridazinyl (e.g., 3-or 4-pyridizinyl); and (3) bicyclic or tricyclic condensed heterocyclic groups containing 1 to 4 hetero atom(s) selected from oxygen atoms, sulfur atoms, nitrogen atoms etc., in addition to carbon atoms, and having a bond in a carbon atom thereof, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolizinyl, indolyl, quinolizinyl, 1,8-naphthylidinyl, purinyl, pteridinyl, dibenzofuranyl, carbazolyl, acridinyl, phenanthridinyl, chromanyl, benzoxazinyl, phenazinyl, phenothiazinyl and phenoxazinyl, and the like.

The substituents in the heterocyclic group having a bond in a carbon atom thereof, which may be substituted is exemplified by the same substituents recited as examples of the above-described heterocyclic group which may be substituted.

The cyclic amino group and the cyclic amino group in the cyclic amino group which may be substituted described above is exemplified by 5- to 7-membered nitrogenitrogen-containing cyclic groups which may have an additional atom selected from oxygen atoms, sulfur atoms and nitrogen atoms. Examples of such groups include pyrrolidinyl, pyrrolinyl, pyrrolyl, pyrazolidinyl, pyrazolinyl, pyrazolyl, imidazolidinyl, imidazolinyl, imidazolyl, 1,2,3-triazinyl, 1,2,3-triazolidinyl, 1,2,3-triazolyl, 1,2,3,4-tetrazolyl, piperidinyl, piperazinyl, azepinyl, hexamethyleneimino, oxazolidino, morpholino, thiazolidino and thiomorpholino. Of these, preferred is 5- to 6-membered cyclic amino group, such as pyrrolidinyl, pyrazolinyl, pyrazolyl, piperidinyl, piperazinyl, morpholino and thiomorpholino.

The cyclic amino group may have 1 to 3 substituent(s) at any substitutable positions, such substituents including, for example, (1) C₁₋₆ alkyl, (2) C₆₋₁₄ aryl, (3) C₇₋₁₀ aralkyl (phenyl-C₁₋₄ alkyl), (4) benzhydryl, (5) C₁₋₆ alkyl-carbonyl, (6) C₆₋₁₄ aryl-carbonyl, (7) C₁₋₆ alkoxy-carbonyl, and the like. Preferred substituent is C₁₋₆ alkyl, more preferred substituent is C₁₋₃ alkyl.

The homocyclic group in the homocyclic group which may be substituted is exemplified by 3- to 7-membered carbocyclic groups which·may be condensed, such as C₆₋₁₀ aryl groups (e.g., phenyl, naphthyl, etc.), C₃₋₇ cycloalkyl groups (e.g., cyclopropyl, cyclobutyl, cyclopentyl, cyclohexyl, cycloheptyl, etc.) and C₃₋₇ cycloalkenyl (e.g., cyclopronyl, cyclobutenyl, cyclopentenyl, cyclohexenyl, cycloheptenyl, etc.), and the like.

Such homocyclic groups may have 1 to 6, preferably 1 to 3, and more preferably 1 or 2, substituent(s) at any substitutable positions. Such substituents include, for example, (1) C₁₋₁₅ alkyl which may be substituted by 1 to 3, preferably 1 or 2, halogen(s), preferably C₁₋₆ alkyl which may be substituted by halogen, (2) C₃₋₁₀ cycloalkyl, (3) C₂₋₁₀ alkenyl, (4) C₂₋₁₀ alkynyl, (5) C₃₋₁₀ cycloalkenyl, (6) C₆₋₁₀ aryl, (7) C₇₋₂₀ aralkyl, (8) nitro, (9) hydroxyl, (10) mercapto, (11) oxo, (12) thioxo, (13) cyano, (14) carbamoyl, (15) carboxyl, (16) C₁₋₆ alkoxy-carbonyl (e.g., methoxycarbonyl, ethoxycarbonyl, etc.), (17) sulfo, (18) halogen, (19) C₁₋₆ alkoxy, (20) C₆₋₁₀ aryloxy (e.g., phenoxy, etc.), (21) C₁₋₆ acyloxy (e.g., C₁₋₆ alkanoyloxy such as acetoxy and propionyloxy), (22) C₁₋₆ alkylthio (e.g., methylthio, ethylthio, n-propylthio, isopropylthio, n-butylthio, t-butylthio, etc.), (23) C₆₋₁₀ arylthio (e.g., phenylthio, etc.), (24) C₁₋₆ alkylsulfinyl (e.g., methylsulfinyl, ethylsulfinyl, etc.), (25) C₆₋₁₀ arylsulfinyl (e.g., phenylsulfinyl, etc.), (26) C₁₋₆ alkylsulfonyl (e.g., methylsulfonyl, ethylsulfonyl, etc.), (27) C₆₋₁₀ arylsulfonyl. (e.g., phenylsulfonyl, etc.), (28) amino, (29) C₁₋₆ acylamino (e.g., C₁₋₆ alkanoylamino such as acetylamino, propionylamino, etc.), (30) mono- or di-C₁₋₄ alkylamino (e.g., methylamino, ethylamino, n-propylamino, isopropylamino, n-butylamino, dimethylamino, diethylamino, etc.), (31) C₃₋₈ cybloalkylamino (e.g., cyclopropylamino, cyclobutylamino, cyclopentylamino, cyclohexylamino, etc.), (32) C₆₋₁₀ arylamino (e.g., anilino, etc.), (33) C₁₋₆ alkanoyl (e.g., formyl, acetyl, hexanoyl, etc.), (34) C₆₋₁₀ aryl-carbonyl (e.g., benzoyl, etc.), and (35) 5- or 6-membered heterocyclic groups containing 1 to 4 hetero atom(s) selected from oxygen, sulfur, nitrogen, etc., in addition to carbon atoms [e.g., thienyl (e.g., 2- or 3-thienyl-), furyl (e.g., 2- or 3-furyl), pyrazolyl (e.g., 3-, 4- or 5-pyrazolyl), thiazolyl (e.g., 2-, 4- or 5-thiazolyl), isothiazolyl (e.g., 3-, 4- or 5-isothiazolyl), oxazolyl (e.g., 2-, 4- or 5-oxazolyl), isoxazolyl (e.g., 3-, 4- or 5-isoxazolyl), imidazolyl (e.g., 2-, 4- or 5-imidazolyl), triazolyl (e.g., 1,2,3- or 1,2,4-triazolyl), tetrazolyl (e.g., 1H- or 2H-tetrazolyl), pyridyl (e.g., 2-, 3- or 4-pyridyl), pyrimidinyl (e.g., 2-, 4- or 5-pyrimidyl), pyridazinyl (e.g., 3- or 4-pyridazinyl), quinolyl, isoquinolyl, indolyl, etc.], and the like.

The hydroxy group which may be substituted for R¹⁸ and R²⁰ includes, for example, a group of the above-mentioned formula: -OR²³ wherein R²³ is as defined above.

In the above formulas, R¹¹, R¹² and R¹³ are the same or different and each is preferably (i) hydrogen or (ii) the above-described group bound via a carbon atom, a nitrogen atom or an oxygen atom. Preference is given to the case wherein R¹¹ is a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a nitro group, a group of the formula: -NR²⁰R²¹ wherein R²⁰ is hydrogen, a C₁₋₁₀ hydrocarbon group which may be substituted, a C₁₋₂₀ acyl group which may be substituted, a hydroxyl group which may be substituted, a heterocyclic group which may be substituted, or a group of the formula: -S(O)t-R²² wherein t is an integer of 0 to 2, and R²² is a hydrogen atom, a C₁₋₁₀ hydrocarbon group which may be substituted or a heterocyclic group which may be substituted; R²¹ is hydrogen or a C₁₋₁₀ hydrocarbon group; or R²⁰ and R²¹ may form, taken together with the adjacent nitrogen atom, a cyclic amino group which may be substituted, or a group of the formula: -O-R²³ wherein R²³ is a hydrogen atom or a C₁₋₁₀ hydrocarbon group which may be substituted, a C₁₋₂₀ acyl group which may be substituted, a C₁₋₂₀ alkylsulfonyl group which may be substituted, a C₆₋₁₄ arylsulfonyl group which may be substituted or a 5- to 8-membered heterocyclic group which may be substituted (same as the above-described "5- to 8-membered heterocyclic group containing 1 to 4 hetero atom(s) selected from oxygen atoms, sulfur atoms, nitrogen atoms, etc., in addition to carbon atoms"), wherein at least one of R¹² and R¹³ is hydrogen, and the other is the above-described group bound via a carbon atom, a nitrogen atom, or an oxygen atom, preferably R¹² and R¹³ are both hydrogens.

R¹¹ is preferably a C₁₋₁₀ alkyl group (preferably a C₁₋₆ alkyl group) which may be substituted by 1 to 3, preferably 1, hydroxyl group, a nitro group, an amino group, the formula: -NR²⁰R²¹ wherein R²⁰ represents hydrogen; R²¹ represents C₁₋₆ alkyl-carbonyl which may be substituted by 1 to 3, preferably 1, hydroxyl group, C₁₋₆ alkylamino-carbonyl or C₆₋₁₄ arylamino-carbonyl), or the formula: -O-R²³ wherein R²³ represents hydrogen, C₁₋₁₀ alkyl which may be substituted by 1 to 3, preferably 1, hydroxyl group, a C₁₋₆ alkyl-carbonyl which may be substituted by C₃₋₁₀ cycloalkyl or 1 to 3, preferably 1, hydroxyl group, a C₁₋₆ alkylsulfonyl group, or a C₆₋₁₀ arylsulfonyl group.

In the above formulas, R¹⁴ is preferably (1) a C₁₋₁₀ hydrocarbon group which may be substituted, (2) a C₁₋₂₀ acyl group which may be substituted, (3) a heterocyclic group having a bond in a carbon atom thereof which may be substituted, (4) a carboxyl group which may be esterified or amidated, or (5) a cyano group. More preferably, R¹⁴ is a C₁₋ ₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted. Still more preferred is a C₁₋₆ alkyl group which may be substituted such as an aminoalkyl group which may be substituted and the like. A preferred example of R¹⁴ is the formula: -(CH₂)n-NR²⁰R²¹ wherein n is an integer of 1 to 3; R²⁰ is hydrogen, a C₁₋₁₀ hydrocarbon group which may be substituted, a C₁₋₂₀ acyl group which may be substituted, a hydroxyl group which may be substituted (group of the above-described formula: -O-R²³), a heterocyclic group which may be substituted, or a group of the formula: -S(O)t-R²² wherein t is an integer of 0 to 2; R²² is a hydrogen atom or a C₁₋₁₀ hydrocarbon group which may be substituted; R²¹ is hydrogen or a C₁₋₁₀ hydrocarbon group; or R²⁰ and R²¹ may form, taken together with the adjacent nitrogen atom, a cyclic amino group which may be substituted. R¹⁴ is more preferably a C₁₋₃ alkyl group which may be substituted by a halogen atom, a hydroxyl group which may be substituted by a C₁₋₂₀ acyl group, or an amino group which may be substituted by C₁₋₁₀ alkyl and/or C₆₋₁₄ aryl-C₁₋₁₀ alkyl. R¹⁴ is particularly preferably N-C₁₋₆ alkyl-N-benzylaminomethyl.

In the above formulas, the halogen represented by R¹⁵ is exemplified by fluoro, chloro, bromo and iodo.

R¹⁵ is preferably hydrogen, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a carboxyl group which may be esterified or amidated, or the formula: -O-R²³ wherein R²³ is a hydrogen atom, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a C₁₋₂₀ alkylsulfonyl group which may be substituted, a C₆₋₁₄ arylsulfonyl group which may be substituted or a heterocyclic group which may be substituted. of these, preferred examples of R¹⁵ include hydrogen, a C₁₋₁₅ alkyl group which may be substituted by 1 to 3, preferably 1 C₆₋₁₄ aryl or C₁₋₆ alkoxy group, or a C₁₋₆ alkyl-carbonyl, C₁₋₆ alkoxycarbonyl (e.g., methoxycarbonyl, ethoxycarbonyl, t-butoxycarbonyl, etc.), C₆₋₁₄ aryl-carbonyl (e.g., benzoyl, etc.), C₆₋₁₄ aryloxy-carbonyl (e.g., phenoxycarbonyl, etc.), C₇₋₁₅ aralkyl-carbonyl (e.g., benzylcarbonyl, etc.), C₇₋₁₉ aralkyloxy-carbonyl (e.g., benzyloxycarbonyl, etc.), N-C₁₋₁₀ alkyl-N-(C₁₋₁₀ alkoxy)amino-carbonyl (e.g., N-methyl-N-methoxyamino-carbonyl, etc.), C₁₋ ₁₅ alkyloxy and C₁₋₂₀ arylsulfonyl group which may be substituted by 1 to 3, preferably 1, hydroxyl group, and the like. R¹⁵ is more preferably (1) a C₁₋₆ alkoxy-carbonyl group, (2) a C₆₋₁₄ aryl group which may be substituted by halogen or C₁₋₆ alkoxy, or (3) a phenyl-C₁₋₃ alkyl group.

In the above formulas, R¹⁶ is preferably hydrogen, a C₁₋ ₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted. More preferably, R¹⁶ is hydrogen or a C₁₋₁₀ alkyl group. Still more preferably, R¹⁶ is hydrogen or a C₁₋₆ alkyl group.

In the above formulas, R¹⁷ is a homocylic group which may be substituted or a heterocyclic group which may be substituted, preferably a C₆₋₁₄ aryl group which may be substituted. More preferably, R¹⁷ is a phenyl group which may be substituted by 1 to 3, preferably 1 or 2, halogen atom(s) or C₁₋₆ alkoxy. Particularly preferred is a phenyl group which may be substituted by 1 or 2 halogen atom(s).

In the above formula (IX), m is 0 to 3, preferably 0 to 2, and more preferably 0 or 1.

In the above formula, n is an integer of 1 to 3, preferably 1 or 2, and more preferably 1.

In the above formula (IX), one of A and D represents a nitrogen atom and the other represents a carbon atom, or both represent nitrogen atom(s); B represents a nitrogen atom or a carbon atom. Compounds represented by the formula (IX) are therefore exemplified by compounds represented by the following formulas: 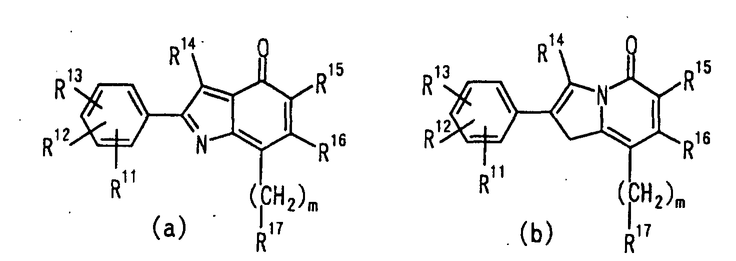 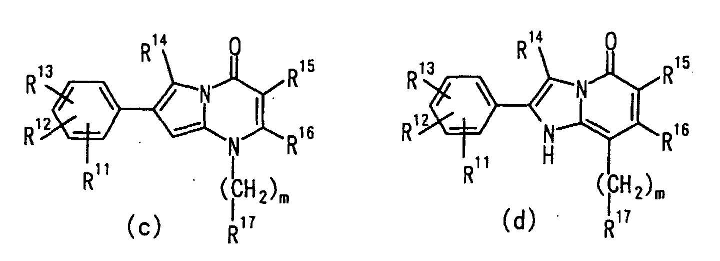 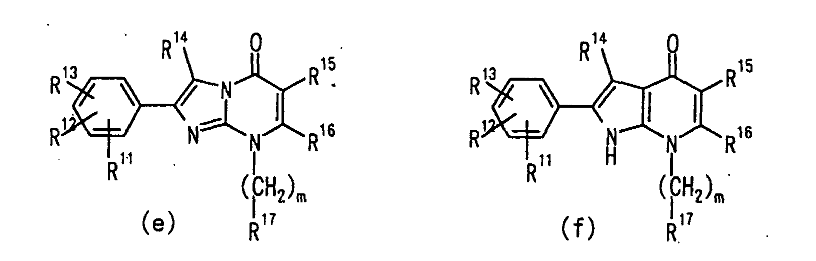 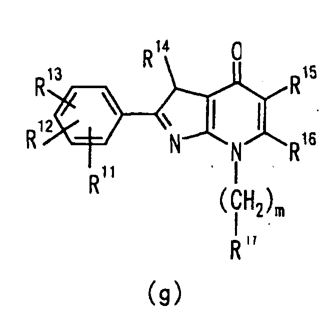 wherein each symbol is as defined above, preferably compounds represented by the formulas (a), (b), (c), (d), (e) or (g). Of these, preferred is a compound of formula (IX) wherein B is a nitrogen atom, particularly preferred is a compound represented by the formulas (c) or (e), and most preferred is a compound represented by the formula (e).

In compound (IX), preferred is a compound of the formula : 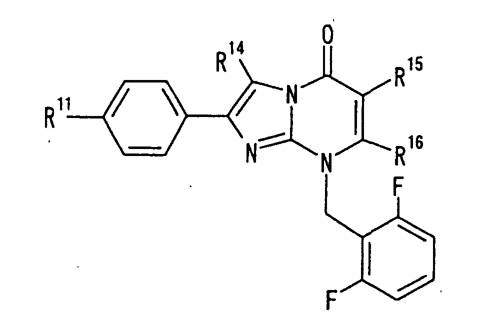 wherein each symbol is as defined above. Of the compound (IX), more preferred is a compound wherein R¹¹ is (1) an amino group which may be substituted by (i) carbamoyl which may be substituted by C₁₋₆ alkyl or C₁₋₆ alkoxy, or (ii) C₁₋₆ alkyl-carbonyl, or (2) a C₁₋₆ alkoxy group which may be substituted by C₃₋₆ cycloalkyl;
R¹⁴ is an N-C₁₋₆ alkyl-N-benzylaminomethyl group;
R¹⁵ is (1) a C₁₋₆ alkoxy-carbonyl group, (2) a C₆₋₁₄ aryl group which may be substituted by halogen or C₁₋₆ alkoxy, or (3) a phenyl-C₁₋₃ alkyl group; and
R¹⁶ is a hydrogen atom.

In addition, a compound wherein R¹¹ is (1) a nitro group, (2) an amino group which may be substituted by 1 or 2 substituent(s) selected from the group consisting of (i) C₁₋₆ alkyl which may be substituted by hydroxy, (ii) C₁₋₆ alkyl-carbonyl which may be substituted by hydroxy, halogen or thienyl, (iii) C₆₋₁₀ aryl-carbonyl which may be substituted by C₁₋₆ alkyl, C₁₋₆ alkoxy or halogen, (iv) C₃₋₆ cycloalkyl-carbonyl, (v) C₂₋₄ alkenyl-carbonyl, (vi) C₁₋₆ alkoxy-carbonyl, (vii) C₁₋₆ alkylamino-carbonyl, (viii) C₁₋₆ alkoxyamino-carbonyl, (ix) phenylaminocarbonyl, (x) isoxazolylcarbonyl, thienylcarbonyl, thiazolylcarbonyl, pyrazolylcarbonyl or furylcarbonyl, which may be substituted by 1 or 2 substituent(s) selected from the group consisting of C₁₋₆ alkyl, nitro and C₁₋₆ alkoxy, (xi) pyridylcarbonyl, (xii) C₁₋₆ alkylsulfonyl, (xiii) thienylsulfonyl and (xiv) phenylsulfonyl which may be substituted by C₁₋₆ alkyl,
(3) a pyrrolyl group or
(4) a hydroxy group which may be substituted by C₁₋₆ alkyl, C₃₋₆ cycloalkyl-C₁₋₃ alkyl or C₁₋₆ alkyl-carbonyl;
R¹⁴ is a C₁₋₆ alkyl group which may be substituted by 1 or 2 substituent(s) selected from the group consisting of (1) halogen, (2) hydroxy and (3) amino which may be substituted by
1 or 2 substituent(s) selected from the group consisting of C₁₋ ₆ alkyl, phenyl-C₁₋₃ alkyl and di-C₁₋₆ alkylamino-C₁₋₃ alkyl; R¹⁵ is (1) halogen, (2) a phenyl group which may be substituted by halogen or C₁₋₆ alkyl, or (3) a carbonyl group substituted
by (i) C₁₋₆ alkyl, (ii) amino substituted by C₁₋₆ alkyl and C₁₋₆ alkoxy or (iii) C₁₋₆ alkoxy; and
R¹⁶ is a hydrogen atom or a C₁₋₃ alkyl group, is also preferable.

As compound (IX), concretely mentioned are 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid ethyl ester,
8-(2,6-difluorobenzyl)-5,8-dihydro-2-[4-(methoxyaminocarbonylamino)phenyl)]-3-(N-methyl-N-benzylaminomethyl)-5-oxoimidazo[1,2-a]pyrimidine-6-carboxylic acid isopropyl ester,
8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid isopropyl ester, salts thereof, and the like.

Compound (IX) may form a salt. The salt is preferably a physiologically acceptable acid addition salt. Such salts include, for example, salts with inorganic acids (e.g., hydrochloric acid, hydrobromic acid, nitric acid, sulfuric acid, phosphoric acid, etc.), physiologically acceptable acid addition salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.) and the like. When compound (IX) of the present invention has an acidic group such as -COOH, it may form a physiologically acceptable salt with an inorganic base (e.g., alkali metals and alkaline earth metals such as sodium, potassium, calcium and magnesium, ammonia, etc.) or an organic base (e.g., trimethylamine, triethylamine, pyridine, picoline, ethanolamine, diethanolamine, triethanolamine, dicyclohexylamine, N,N'-dibenzylethylenediamine, etc.).

Also, the compound (IX) or a salt thereof may be a hydrate or a non-hydrate. The hydrate is exemplified by monohydrate, sesquihydrate and dihydrate, and the like.

Compound (IX) or a salt thereof can be produced according to the method described in JP-A-11-315079.

In another embodiment of the present invention, the present invention provides an agent for the prophylaxis or treatment of Alzheimer's disease, containing a non-peptide compound that lowers LH and/or RH (preferably an agent for the prophylaxis or treatment of Alzheimer's disease, containing a non-peptide compound that lowers LH and RH).

The non-peptide compound that lowers LH and/or RH is exemplified by the aforementioned "compound having a gonadotropin releasing hormone (GnRH)-antagonistic action" (GnRH antagonist).

The compound having a GnRH antagonistic action, and a non-peptide compound that lowers LH and/or RH have low toxicity.

The compound having a GnRH antagonistic action or a non-peptide compound that lowers LH and/or RH is prepared into a pharmaceutical composition according to a method known *per se,* and can be administered orally or parenterally to a mammal (e.g., human, monkey etc.) suffering from Alzheimer's disease (Alzheimer's disease, senile dementia of Alzheimer type and a mixed type thereof) in various dosage forms.

To be specific, a compound having a GnRH antagonistic action, or a non-peptide compound that lowers LH and/or RH is admixed with a pharmaceutically acceptable carrier and generally formulated into solid preparations such as tablets, capsules, granules and powders for oral administration, or into intravenous, subcutaneous, intramuscular or other injections, suppositories or sublingual tablets, etc. for parenteral administration. It may also be sublingually, subcutaneously or intramuscularly administered in the form of sustained-release preparations such as sublingual tablets, and microcapsules and the like.

The above pharmaceutically acceptable carriers are various organic or inorganic carrier substances in common use as pharmaceutical materials, including excipients, lubricants, binders and disintegrants for solid preparations; and solvents, dissolution aids, suspending agents, isotonizing agents, buffers and soothing agents for liquid preparations, and the like. Other pharmaceutical additives such as preservatives, antioxidants, coloring agents and sweetening agents may be used as necessary.

Preferable excipients above include, for example, lactose, sucrose, D-mannitol, starch, crystalline cellulose and light silicic anhydride and the like. Preferable lubricants above include, for example, magnesium stearate, calcium stearate, talc and colloidal silica and the like. Preferable binders above include, for example, crystalline cellulose, sucrose, D-mannitol, dextrin, hydroxypropylcellulose, hydroxypropylmethylcellulose and polyvinylpyrrolidone and the like. Preferable disintegrants above include, for example, starch, carboxymethylcellulose, carboxymethylcellulose calcium, crosscarmellose sodium, sodium carboxymethyl starch and the like. Preferable solvents above include, for example, water for injection, alcohol, propylene glycol, macrogol, sesame oil, corn oil and the like. Preferable dissolution aids above include, for example, polyethylene glycol, propylene glycol, D-mannitol, benzyl benzoate, ethanol, trisaminomethane, cholesterol, triethanolamine, sodium carbonate, sodium citrate and the like. Preferable suspending agents above include, for example, surfactants such as stearyltriethanolamine, sodium lauryl sulfate, laurylaminopropionic acid, lecithin, benzalkonium chloride, benzethonium chloride, glyceryl monostearate and the like; and hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, and the like. Preferable isotonizing agents above include, for example, sodium chloride, glycerol, D-mannitol and the like. Preferable buffers above include, for example, buffer solutions of phosphates, acetates, carbonates, citrates etc., and the like. Preferable soothing agents include, for example, benzyl alcohol and the like. Preferable preservatives above include, for example, p-hydroxybenzoic acid esters, chlorobutanol, benzyl alcohol, phenethyl alcohol, dehydroacetic acid, sorbic acid and the like. Preferable antioxidants above include, for example, sulfites, ascorbic acid and the like.

Depending on symptom severity; subject age, sex and weight; duration and intervals of administration; property, dispensing and kind of pharmaceutical preparation; kind of active ingredient etc., the daily dose varies and is not subject to any particular limitation. For oral use in the treatment of Alzheimer's disease, the dose is generally 0.1-300 mg, preferably about 1 - 300 mg, more preferably about 10-200 mg, per day for an adult, which is generally administered once to four times daily.

The content of the compound having a GnRH antagonistic action, or a non-peptide compound that lowers LH and/or RH in the agent of the present invention is about 0.01 to 100 wt% of the agent as a whole.

The compound having a GnRH antagonistic action and a non-peptide compound that lowers LH and/or RH can be used in combination with, for example, a central pharmaceutical agent [e.g., antianxiety, sleep inducing agent, schizophrenia therapeutic agent, therapeutic agent of Parkinson's disease, anti-dementia (e.g., cerebral circulation improver, brain metabolism activator and the like) and the like], antihypertensive agent, therapeutic agent of diabetes, anti-hyperlipidemia agent, nutrient preparation (e.g., vitamins and the like), digestibility promoter, gastrointestinal drug and the like.

In addition, the compound having a GnRH antagonistic action and a non-peptide compound that lowers LH and/or RH can be used in combination with acetylcholinesterase inhibitor (e.g., tacrine, donepezil, rivastigmine, galantamine, physostigmine-DDS, ipidacrine etc.), muscarinic acetylcholine receptor agonist, nicotinic acetylcholine receptor agonist, Ca antagonist (e.g., nimodipine etc.), COX-2 inhibitor (e.g., rofecoxib, celecoxib etc.), AMPA receptor agonist, monoamine oxidase inhibitor (e.g., selegiline-DDS), amyloid β protein secretion-coagulation inhibitor, or a therapeutic agent of dementia of Alzheimer type such as nifiracetam, and Memantine.

The present invention is hereinafter described in more detail by means of, but is not limited to, the reference examples and examples.

¹H-NMR spectra are determined with tetramethylsilane as the internal standard, using the varian GEMINI 200 (200 MHz) spectrometer, the JEOL LAMBDA 300 (300 MHz) spectrometer or the Bruker AM500 (500 MHz) spectrometer; all δ values are shown in ppm. Unless otherwise specifically indicated, "%" is by weight. Yield indicates mol/mol %. The other symbols used herein have the following definitions:
s: singlet
d: doublet
t: triplet
dt: double triplet
m: multiplet
br: broad

The term "room temperature" indicates the range from about 15 to 25°C, but is not to be construed as strictly limitative.

### Examples

### Reference Example 1

### Ethyl 2-amino-4-methyl-5-(4-nitrophenyl)thiophene-3-carboxylate

A mixture of 4-nitrophenylacetone (35.0 g, 195 mmol), ethyl cyanoacetate (23.8 g, 195 mmol), ammonium acetate (3.1 g, 40 mmol) and acetic acid (9.1 ml, 159 mmol) was heated on reflux for 24 hours, with removing water produced with a Dean-Stark trap. After cooling, the reaction mixture was concentrated under reduced pressure and the residue was partitioned between dichloromethane and aqueous sodium hydrogencarbonate solution. The organic layer was washed with aqueous sodium chloride solution and dried (MgSO₄) and the solvent was distilled off under reduced pressure. The residue was chromatographed on silica gel to give oil compound. The oil thus obtained was dissolved in ethanol followed by addition of sulfur (5.0 g, 160 mmol) and diethylamine (16.0 ml, 160 mmol), and the mixture was stirred at 60 to 70°C for 2 hours. After cooling, the reaction mixture was concentrated under reduced pressure to yield residue, which was partitioned between dichloromethane and aqueous sodium hydrogencarbonate solution. The organic layer was washed with aqueous sodium chloride solution and dried (MgSO₄) and the solvent was distilled off under reduced pressure. The residue was chromatographed on silica gel to give the crude product, which was crystallized from ether-hexane to give the title compound as red plates (22.2 g, 52%). mp: 168-170°C (recrystallized from ether-hexane).

| Elemental analysis for C₁₄H₁₄N₂O₄S | | | |
|---|---|---|---|
| | C (%) | H (%) | N (%) |
| Calculated | 54.89 ; | 4.61 ; | 9.14 |
| Found | 54.83 ; | 4.90 ; | 9.09 |

¹H-NMR (200MHz, CDCl₃) δ: 1.39 (3H, t, J=7.1Hz), 2.40 (3H, s), 4.34 (2H, q, J=7.1Hz), 6.27 (2H, br), 7.48 (2H, d, J=8.7Hz), 8.23 (2H, d, J=8.7Hz).
IR (KBr): 3446, 3324, 1667, 1580, 1545, 1506, 1491, 1475, 1410, 1332 cm⁻¹.

### Reference Example 2

### 5-Methyl-6-(4-nitrophenyl)-3-phenylthieno[2,3-d]pyrimidine-2,4(1H,3H)-dione

To a solution of the compound obtained in Reference Example 1 (5.00 g, 16.32 mmol) in pyridine (30 ml) was added phenyl isocyanate (2.66 ml, 24.48 mmol). After 6 hours of stirring at 45°C, the reaction mixture was concentrated under reduced pressure and the residue thus obtained was dissolved in ethanol (6 ml). To this solution was added 28% sodium methoxide (7.86 g, 40.80 mmol), and the reaction mixture was stirred at room temperature for 2 hours. Then, 2N-hydrochloric acid (25 ml, 50 mmol) was added and the solvent ethanol was distilled off under reduced pressure. The obtained residue was filtered, washed with water-ethanol, dried under reduced pressure, and recrystallized from ethanol to give the title compound as yellow powder (6.09 g, 98%).
mp: >300°C.

| Elemental analysis for C₁₉H₁₃N₃O₄S·0.3H₂O | | | |
|---|---|---|---|
| | C(%) | H(%) | N(%) |
| Calculated | 59.30 ; | 3.56 ; | 10.92 |
| Found : | 59.56 ; | 3.52 ; | 10.93 |

¹H-NMR (300MHz, DMSO-d₆) δ: 2.50 (3H, s), 7.31-7.46 (5H, m), 7.78 (2H, d, J=8.8Hz), 8.32 (2H, d, J=8.8Hz), 12.50 (1H, s).
IR (KBr): 1715, 1657, 1593, 1510 cm⁻¹.

### Reference Example 3

### 1-(2,6-Difluorobenzyl)-5-methyl-6-(4-nitrophenyl)-3-phenylthieno[2,3-d]pyrimidine-2,4(1H,3H)-dione

To a solution of the compound obtained in Reference Example 2 (52.54 g, 0.131 mol) in dimethylformamide (1.0 l) were added potassium carbonate (19.00 g, 0.138 mol), potassium iodide (22.90 g, 0.138 mol) and 2,6-difluorobenzyl chloride (22.40 g, 0.138 mol), and the mixture was stirred at room temperature for 2 hours. This reaction mixture was concentrated to give the residue, which was partitioned between chloroform and aqueous sodium chloride solution. The aqueous layer was extracted with chloroform. The combined extracts were washed with aqueous sodium chloride solution and dried (MgSO₄) and the solvent was distilled off under reduced pressure. The residue thus obtained was chromatographed on silica gel to give the title compound as light-yellow crystals (61.50 g, 93%).
mp: 280-282°C.

| Elemental analysis for C₂₆H₁₇N₃O₄SF₂ | | | |
|---|---|---|---|
| | C(%) | H(%) | N(%) |
| Calculated | 61.78 ; | 3.39 ; | 8.31 |
| Found | 61.67 ; | 3.46 ; | 8.21 |

¹H-NMR (300MHz, CDCl₃) δ: 2.57 (3H, s), 5.38 (2H, s), 6.94 (2H, d, J=8.1Hz), 7.42-7.58 (8H, m), 8.29 (2H, d, J=8.8Hz).
IR (KBr): 1719, 1669, 1524, 1473 cm⁻¹.

### Reference Example 4

### 5-Bromomethyl-1-(2,6-difluorobenzyl)-6-(4-nitrophenyl)-3-phenylthieno[2,3-d]pyrimidine-2,4(1H,3H)-dione

A mixture of the compound obtained in Reference Example 3 (30.34 g, 0.060 mol), N-bromosuccinimide (12.81 g, 0.072 mol), α,α'-azobisisobutyronitrile (1.15 g, 0.007 mol) and chlorobenzene (450 ml) was stirred at 85°C for 3 hours. After cooling, the reaction mixture was washed with aqueous sodium chloride solution and dried (MgSO₄) and the solvent was then distilled off under reduced pressure. The residue thus obtained was recrystallized from ethyl acetate to give the title compound as yellow needles (80.21 g, 100%).
mp: 228-229°C.
¹H-NMR (300MHz, CDCl₃) δ: 4.77 (2H, s), 5.38 (2H, s), 6.96 (2H, t, J=8.1Hz), 7.29-7.58 (6H, m), 7.79 (2H, d, J=8.5Hz), 8.35 (2H, d, J=8.5Hz).
IR (KBr): 1721, 1680, 1524, 1473, 1348 cm⁻¹.
FAB-Mass m/z 584(MH)⁺

### Reference Example 5

### 5-(N-Benzyl-N-methylaminomethyl)-1-(2,6-difluorobenzyl)-6-(4-nitrophenyl)-3-phenylthieno[2,3-d]pyrimidine-2,4(1H,3H)-dione

To a solution of the compound obtained in Reference Example 4 (80.00 g, 0.119 mol) in dimethylformamide (600 ml) were added ethyldiisopropylamine (27.00 ml, 0.155 mol) and benzylmethylamine (18.45 ml, 0.143 mol) with ice-cooling. After 2 hours of stirring at room temperature, the reaction mixture was concentrated and the residue thus obtained was partitioned between ethyl acetate and saturated aqueous sodium hydrogencarbonate solution. The aqueous layer was extracted with ethyl acetate. The organic layers were combined and dried (MgSO₄) and the solvent was distilled off under reduced pressure. The residue thus obtained was chromatographed on silica gel to give a yellow oil (74.90 g, 100%), which was recrystallized from ethyl acetate to give the title compound as yellow needles.
mp: 173-174°C.

| Elemental analysis for C₃₄H₂₆N₄O₄SF₂·0.5H₂O | | | |
|---|---|---|---|
| | C(%) | H(%) | N(%) |
| Calculated | 64.45 ; | 4.29 ; | 8.84 |
| Found | 64.50 ; | 4.24 ; | 8.82 |

¹H-NMR (300MHz, CDCl₃) [Free amine] δ: 1.31 (3H, s), 3.60 (2H, s), 3.96 (2H, s), 5.39 (2H, s), 6.95 (2H, t, J=8.2Hz), 7.18-7.55 (11H, m), 8.02 (2H, d, J=9.0Hz), 8.26 (2H, d, J=9.0Hz).
IR (KBr) [Hydrochloride]: 1719, 1678, 1597, 1520 cm⁻¹.

### Reference Example 6

### 6-(4-Aminophenyl)-5-(N-benzyl-N-methylaminomethyl)-1-(2,6-difluorobenzyl)-3-phenylthieno[2,3-d]pyrimidine-2,4(1H,3H)-dione

To a solution of the compound obtained in Reference Example 5 (3.00 g, 4.80 mmol) in formic acid (30 ml) were added 1M hydrogen chloride - ether (14.4 ml, 14.4 mmol) and 10% palladium-on-carbon powder (300 mg) with ice-cooling, and hydrogenation was carried out under atmospheric condition at room temperature with stirring for 2 hours. This reaction mixture was filtered through Celite and the filtrate was concentrated under reduced pressure. The residue thus obtained was partitioned between dichloromethane and saturated aqueous sodium hydrogencarbonate solution. The aqueous layer was extracted with dichloromethane and the organic layers were combined and dried (MgSO₄). The solvent was then distilled off under reduced pressure. The residue thus obtained was chromatographed on silica gel to give the title compound as white crystals (2.41 g, 84%).
mp: 205-207°C.

| Elemental analysis for C₃₄H₂₈N₄O₂SF₂·0.1AcOEt·1.2H₂O | | | |
|---|---|---|---|
| | C(%) | H(%) | N(%) |
| Calculated | 66.09 ; | 5.03 ; | 8.96 |
| Found | 66.93 ; | 4.94 ; | 8.67 |

¹H-NMR (300MHz, CDCl₃) δ: 2.05(3H, s), 3.56(2H, s), 3.83(2H, br), 3.88(2H, s), 5.36(2H, s), 6.70(2H, d, J=8.8Hz), 6.88-6.94(2H, m), 7.21-7.31(8H, m), 7.41-7.53(5H, m).
IR (KBr): 1715, 1657, 1628, 1537 cm⁻¹.

### Reference Example 7

### 5-(N-Benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione

To a solution of the compound (5.0 g, 8.41 mmol) obtained in Reference Example 6 in dichloromethane (120 ml) was added triethylamine (2.34 ml, 16.82 mmol) under ice-cooling, and the mixture was stirred. To the reaction mixture was added N,N'-carbonyldiimidazole (2.73 g, 16.82 mmol) under ice-cooling and the mixture was allowed to warm from under ice-cooling to room temperature. The mixture was stirred for 42 hours. The mixture was returned to under ice-cooling, and O-methylhydroxylamine hydrochloride (7.02 g, 84.08 mmol) and triethylamine (11.7 ml, 84.08 mmol) were added. The reaction mixture was allowed to warm from under ice-cooling to room temperature and stirred for 3 hours. The reaction mixture was partitioned between chloroform and saturated aqueous sodium hydrogencarbonate solution. The aqueous layer was extracted with chloroform, the extracts were combined, washed with brine and dried over MgSO₄, after which the solvent was evaporated under reduced pressure. The obtained residue was purified by silica gel column chromatography to give a pale-yellow solid, which was recrystallized from chloroform-ether to give the title compound as white crystals (4.52 g, 80%).
mp: 204-205°C.

| elemental analysis for C₃₆H₃₁N₅O₄SF₂ | | | |
|---|---|---|---|
| | C(%) | H(%) | N(%) |
| Calculated | 64.75; | 4.68; | 10.49 |
| Found | 64.61; | 4.67; | 10.31 |

¹H-NMR (300MHz, CDCl₃) δ: 2.05(3H,s), 3.57(2H,s), 3.82(3H,s), 3.90(2H,s), 5.37(2H,s), 6.92(2H,d,J=8.2Hz), 7.16-7.31(9H,m), 7.42-7.57(5H,m), 7.63(1H,s), 7.73(2H,d,J=8.8Hz).
IR(KBr): 3338, 3064, 1717, 1669, 1628, 1591, 1531, 1470cm⁻¹.

### Reference Example 8

### 3-(N-Benzyl-N-methylaminomethyl)-4,7-dihydro-5-isobutyryl-7-(2,6-difluoroberizyl)-2-[4-[(1-hydroxycyclopropyl)-carbonylamino]phenyl]-4-oxothieno[2,3-b]pyridine

To a solution of 2-(4-aminophenyl)-7-(2,6-difluorobenzyl)-4,7-dihydro-5-isobutyryl-3-(N-benzyl-N-methylaminomethyl)-4-oxothieno[2,3-b]pyridine (0.57 g, 1.0 mmol) in dichloromethane (10 ml) were added diisopropylethylamine (0.52 g, 4 mmol) and 2-hydroxycyclopropanecarboxylic acid (0.204 g, 2 mmol) and the mixture was stirred under ice-cooling. To this solution was added benzotriazole-1-yloxytrisdimethylaminophosphonium hexafluorophosphate (BOP reagent)(1.76 g, 4 mmol). The mixture was stirred under ice-cooling for 1 hour and further at room temperature for 4 days. The reaction mixture was concentrated to dryness under reduced pressure, and the obtained residue was partitioned between water (50 ml) and chloroform (50 ml). The aqueous layer was again extracted with chloroform (10 ml), the extracts were combined, washed with brine and dried over MgSO₄, after which the solvent was evaporated under reduced pressure. The obtained residue was purified by silica gel column chromatography and recrystallized from ether to give yellow powder crystals (0.27 g, 41%).
¹H-NMR (300MHz, CDCl₃) δ: 1.16-1.20(2H,m), 1.18(6H,d), 1.48-1.51(2H,m), 2.09(3H,s), 3.64(2H,s), 3.95(1H,br s), 4.14(2H,s), 4.12-4.19(1H,m), 5.20(2H,s), 6.99(2H,t), 7.10-7.25(5H,m), 7.34-7.46(1H,m), 7.57(2H,d), 7.70(2H,d), 8.21(1H,s), 8.82(1H, s).

### Example 1

Using the compound (100 mg) produced in Reference Example 7, lactose (165 mg), cornstarch (25 mg), polyvinyl alcohol (4 mg) and magnesium stearate (1 mg), tablets were produced according to the conventional method.

### Example 2

Using the compound (100 mg) produced in Reference Example 8, lactose (165mg), cornstarch(25 mg), polyvinyl alcohol (4 mg) and magnesium stearate (1 mg), tablets were produced according to the conventional method.

### Experimental Example 1

### Suppression of blood LH in castrated monkeys

3-(N-Benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine hydrochloride was orally administered to castrated male cynomolgus monkeys (Macaca fascicularis), and blood LH was quantified. The male cynomolgus monkeys, used at 3 years 8 months to 7 years 7 months of age at the time of experimentation, had been castrated more than 6 months prior to the experimentation. Test animals [n = 2] were given 30 mg/kg (3 ml/kg) of the compound suspended in 0.5% methylcellulose at a final concentration of 1% by oral administration, and control test animals [n = 3] were given 3 ml/kg of the 0.5% methylcellulose dispersant alone by oral administration. At 24 hours and immediately before administration and at 2, 4, 6, 8, 24 and 48 hours after administration, blood was collected for heparinized plasma samples via the femoral vein and immediately stored under freezing conditions.

Plasma LH concentrations were determined by a bioassay using mouse testicular cells. The testicular cells were collected from male BALB/c mice (8 to 9 weeks of age) and washed three times with 1 ml of Dulbecco's modified Eagle medium (DMEM-H) containing 20 mM HEPES and 0.2% BSA per testis. After incubation at 37°C for 1 hour, the cells were passed through a nylon mesh (70 µm) and dispensed at 8 x 10⁵ cells/tube. After the cells were washed twice with 0.4 ml of DMEM-H, 0.4 ml of a DMEM-H solution containing either equine LH (Sigma), as the standard LH, or monkey plasma, ultimately diluted up to 100 fold, as the test sample, was added, followed by a reaction at 37°C for 2 hours. The testosterone concentration in the culture supernatant was determined by a radioimmunoassay (CIS Diagnostics), and the LH concentration in the test monkey plasma was calculated from the standard curve for the standard equine LH.

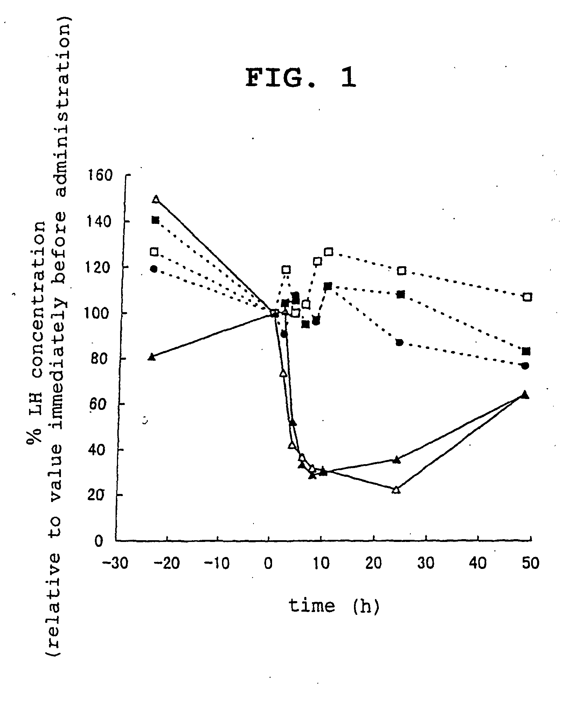

The results are given together in Figure 1. The compound means 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine hydrochloride. In the Figure, controls (1), (2) and (3) show the time course changes in the percentage (%) of the LH concentration of each control test animal (cynomolgus monkey) relative to the baseline LH concentration immediately before administration in each animal. Similarly, compounds (1) and (2) show the time course changes in the percentage (%) of each animal (cynomolgus monkey) administered with 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine hydrochloride relative to the baseline values, wherein the administration time being taken as 0, and the values before and after administration being indicated as the time course by the minus and plus signs, respectively.

### Experimental Example 2

### Suppression of blood LH in castrated monkeys

5-(N-Benzyl-N-methylaminomethyl)-1-(2,6-difluorobenzyl)-6-[4-(3-methoxyureidb)phenyl]-3-phenylthieno[2,3-d]pyrimidine-2,4(1H,3H)-dione hydrochloride was orally administered to castrated male cynomolgus monkeys (Macaca fascicularis), and blood LH was quantified. The male cynomolgus monkeys, used at 4 years 9 months to 6 years 3 months of age at time of experimentation, had been castrated more than 3 months prior to the examination. Test animals [n = 3] were given 30 mg/kg (3 ml/kg) of 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione hydrochloride suspended in 0.5% methylcellulose at a final concentration of 1% by oral administration, and control test animals [n = 2] were given 3 ml/kg of the 0.5% methylcellulose dispersant alone by oral administration. At 24 hours and immediately before administration and at 2, 4, 6, 8, 24, and 48 hours after administration, blood was collected for heparinized plasma samples via the femoral vein and immediately stored under freezing conditions.

Plasma LH concentrations were determined by a bioassay using mouse testicular cells. The testicular cells were collected from male BALB/c mice (8 to 9 weeks of age) and washed three times with 1 ml of Dulbecco's modified Eagle medium (DMEM-H) containing 20 mM HEPES and 0.2% BSA per testis. After incubation at 37°C for 1 hour, the cells were passed through a nylon mesh (70 µm) and dispensed at 8 x 10⁵ cells/tube. After the cells were washed twice with 0.4 ml of DMEM-H, 0.4 ml of a DMEM-H solution containing either equine LH (Sigma), as the standard LH, or monkey plasma, finally diluted up to 300 fold, as the test sample, was added, followed by a reaction at 37°C for 2 hours. The testosterone concentration in the culture supernatant was determined by a radioimmunoassay (CIS Diagnostics), and the LH concentration in the test monkey plasma was calculated from the standard curve for the standard equine LH.

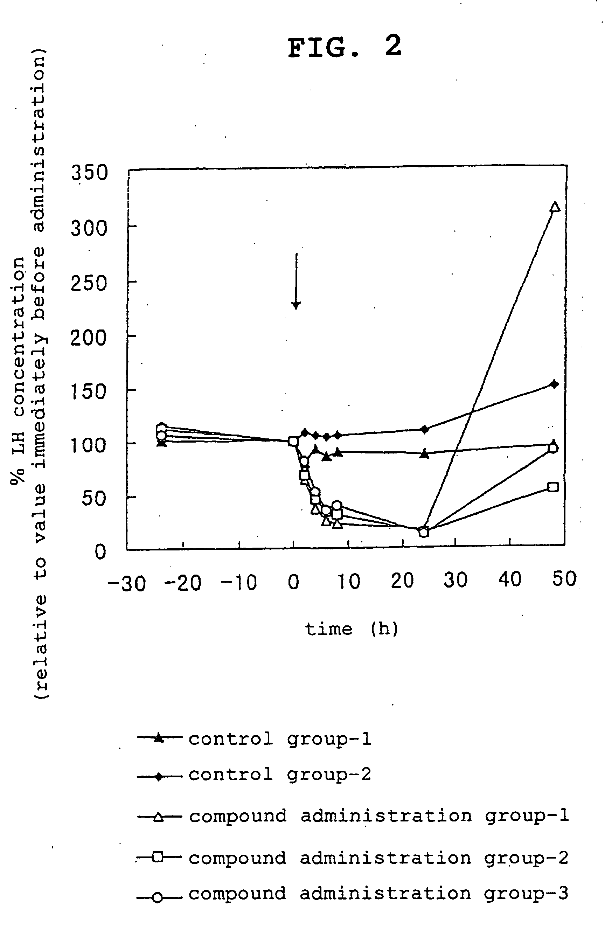

The results are given together in Figure 2.

The LH concentration is expressed in the percentage (%) relative to the LH concentration immediately before administration in each individual test cynomolgus monkey and is shown as the time course with the administration time being taken as 0 (indicated by the arrow mark) and values before and after administration being indicated by the minus and plus signs, respectively. The control group-1 (-▲-) and control group-2 (-◆-) orally received 0.5% methylcellulose dispersant (3 ml/kg) only, while the compound administration group-1 (-Δ-), compound administration group-2 (-□-) and compound administration group-3 (-○-) orally received a dispersion of 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione hydrochloride in 0.5% methylcellulose (30 mg/kg, 3 ml/kg).

The control groups showed little change in the blood LH concentration even after administration. On the other hand, in the compound administration groups, the blood LH concentration showed a rapid fall beginning immediately after administration and had fallen to 20% or less of the value immediately before administration, in 24 hours after administration. Then, at 48 hours after administration, re-elevation of the blood LH concentration was noted.

The above results indicate that the 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione hydrochloride administered orally, has a significant decreasing effect on blood LH concentration.

It is evident from the foregoing results that the above-mentioned compound having a GnRH-antagonistic action blocks the LH-RH stimulation from the hypothalamus to inhibit the LH release, by antagonizing the pituitary LH-RH receptors.

### Industrial Applicability

The agent for the prophylaxis or treatment of Alzheimer's disease of the present invention shows low toxicity, and has a superior preventive and therapeutic effect on Alzheimer's disease.

## Claims

1. An agent for the prophylaxis or treatment of Alzheimer's disease, which comprises a compound having a gonadotropin releasing hormone antagonistic action.

2. The agent of claim 1, wherein the compound is a non-peptide compound.

3. The agent of claim 1, wherein the compound is a fused heterocyclic compound.

4. The agent of claim 1, wherein the compound is represented by the formula: 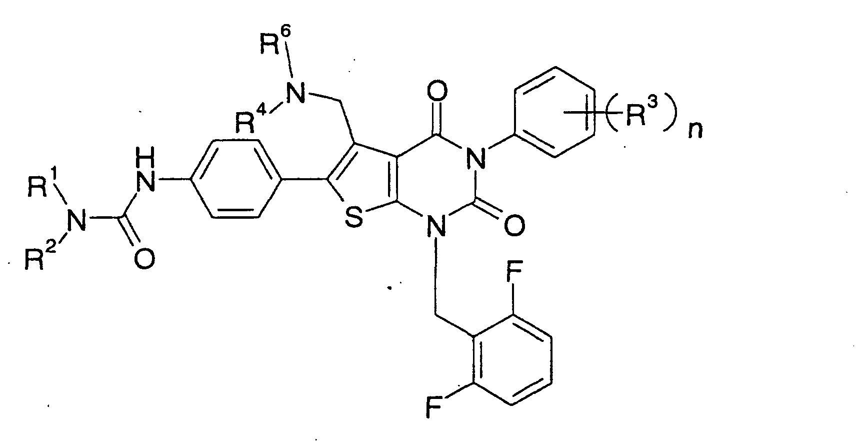 wherein R¹ and R² each represents a hydrogen atom, a hydroxy group, a C₁₋₄ alkoxy group, a C₁₋₄ alkoxy-carbonyl group or a C₁₋₄ alkyl group which may be substituted;
R³ represents a hydrogen atom, a halogen atom, a hydroxy group or a C₁₋₄ alkoxy group which may be substituted; or adjacent two R³ may form, taken together, a C₁₋₄ alkylenedioxy group;
R⁴ represents a hydrogen atom or a C₁₋₄ alkyl group;
R⁶ represents a C₁₋₄ alkyl group which may be substituted or a group of the formula: 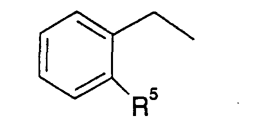 wherein R⁵ represents a hydrogen atom or R⁴ and R⁵ may form, taken together, a heterocycle; and
n represents an integer of 0 to 5; or a salt thereof.

5. The agent of claim 1, wherein the compound is 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof.

6. The agent of claim 1, wherein the compound is represented by the formula: 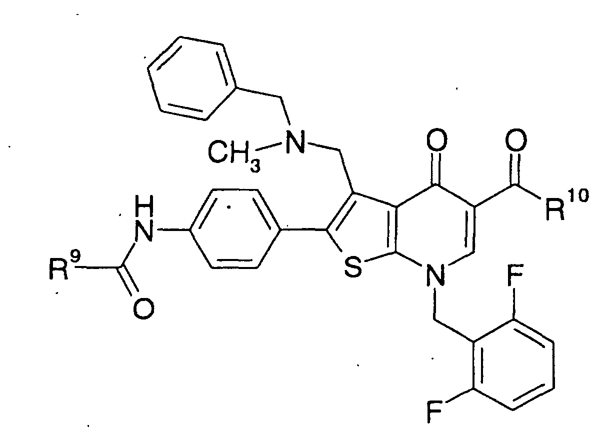 wherein R⁹ represents a C₁₋₇ alkyl group which may be substituted, a C₃₋₇ cycloalkyl group which may be substituted, a C₁₋₆ alkoxyamino group which may be substituted or a hydroxyamino group which may be substituted; and
R¹⁰ represents a C₁₋₇ alkyl group which may be substituted or a phenyl group which may be substituted;
when R⁹ is an unsubstituted C₁₋₇ alkyl group, then R¹⁰ is a substituted C₁₋₇ alkyl group or substituted phenyl,
or a salt thereof.

7. The agent of claim 1, wherein the compound is 3-(N-benzyl-N-methylaminomethyl)-4,7-dihydro-5-isobutyryl-7-(2,6-difluorobenzyl)-2-[4-[(1-hydroxycyclopropyl)carbonylamino]-phenyl]-4-oxothieno[2,3-b]pyridine or a salt thereof.

8. An agent for the prophylaxis or treatment of Alzheimer's disease, which comprises a non-peptide compound that lowers LH and/or RH.

9. An agent for the prophylaxis or treatment of Alzheimer's disease, which comprises a non-peptide compound that lowers LH and RH.
